# EUROPEAN PATENT APPLICATION

(11) **EP 4 269 442 A1**
(43) Date of publication of application: **01.11.2023**
(21) Application number: 21914286.6
(22) Date of filing: 27.12.2021
(51) Int. Cl.: C07K 16/30, C12N 15/13, G01N 33/574, G01N 33/68, C12N 7/01, A61K 39/395, A61P 35/00, C12R 1/92

(54) **MESOTHELIN BINDING MOLECULE AND APPLICATION THEREOF**

(30) Priority: 28.12.2020 CN 202011582948; 28.12.2020 CN 202011584014
(71) Applicant: Zhejiang Nanomab Technology Center Co. Ltd., Changle Township Shengzhou Shaoxing City Zhejiang Province, 312467 (CN)
(72) Inventor: LI, Jiaguo, Shanghai 201805 (CN); YU, Haixiang, Shanghai 201805 (CN); LIU, Xiangzhen, Shanghai 201805 (CN); ZHU, Weimin, Danville, California 94506 (US); SUN, Yan, Shanghai 201805 (CN); DING, Na, Shanghai 201805 (CN); QIAN, Qijun, Shanghai 201805 (CN)
(74) Representative: Cabinet Beau de Loménie
(86) International application number: PCT/CN2021/141734
(87) International publication number: WO 2022/143550

(57) **Abstract**

The present invention provides a mesothelin binding molecule, comprising an anti-mesothelin single domain antibody. Complementarity determining regions (CDR) of the single domain antibody comprise CDR1 shown in SEQ ID NO: 1, CDR2 shown in SEQ ID NO: 2, and CDR3 shown in SEQ ID NO: 3.

## Description

### Technical Field

The invention relates to the field of biomedicine or biopharmacy, more specifically to a mesothelin binding molecule and use thereof.

### Background

The mesothelin (MSLN) gene is located on chromosome 1p13.3, with a total length of 8kD. The gene contains an open reading frame of 1884bp, encoding 17 exons and 628 amino acids. The precursor protein of MSLN is a glycoprotein with a length of about 69kD anchored to the cell membrane by the glycosyl phospholipid peptide inositol, which can be hydrolyzed into two parts by a protease, wherein the N-terminal is a 31kD soluble protein with megakaryocyte stimulating activity, which is known as megakaryocyte potentiating factor (MPF); and the C-terminus is a 40kD membrane-bound protein with cell adhesion, which is called MSLN. The structure of MSLN in membrane protein can be divided into three different segments, wherein segment I is the binding site for ligand CA125.

Mesothelin, as a differentiation antigen, is highly expressed in a variety of malignant tumors and is closely related to the occurrence and development of tumors. This target is one of the most potential anti-tumor targets. Many large pharmaceutical companies have targeted this target for development. Currently, the drug forms entering clinical trials include CAR-T, monoclonal antibody, ADC, etc. In the past 20 years, a variety of monoclonal antibodies against mesothelin have been developed, mainly including SS1P immunotoxin and MORAB-009. SS1P is a recombinant immunotoxin composed of scFv fused with truncated pseudoextracellular toxin, which mainly mediates cell killing; and MORAB-009 is an IgG1 antibody formed by recombining SS1P sequence, which mainly initiates antibody dependent cell-mediated cytotoxicity (ADCC).

SS1P antibody binds to the segment I of mesothelin and can block the binding of CA125 to mesothelin. This antibody has also been used in the treatment of CAR-T, but the efficacy is not ideal (Jiang, H., et al., Protein Cell 8, 926-931, 2017; Adusumilli, P. S. et. al., Sci. Transl. Med. 6, 261ra151, 2014; Lanitis, E. et. al., Mol. Ther. 20, 633-643, 2012).

Nanoantibodies have natural advantages of high stability, strong penetration and wide range of binding epitopes (Muyldermans S. Annu Rev Biochem. 2013; 82: 775-97). There is little research on nanoantibodies targeting the proximal membrane end of MSLN. It has become an urgent problem to develop a novel anti-mesothelin nanoantibody, which has good specificity, blocking activity, clinical efficacy, simple production, low cost, and reduced drug burden.

### Summary

The invention aims to provide a novel anti-mesothelin binding molecule and use thereof.

The first aspect of the invention provides a mesothelin binding molecule, which comprises an anti-mesothelin single domain antibody, wherein the complementarity determining region (CDR) of the single domain antibody comprises CDR1, CDR2 and CDR3, wherein CDR1 includes the sequence shown in SEQ ID NO: 1, CDR2 includes the sequence shown in SEQ ID NO: 2, and CDR3 includes the sequence shown in SEQ ID NO: 3.

In one or more embodiments, SEQ ID NO: 1 is X₁X₂X₃X₄X₅X₆X₇X₈X₉X₁₀X₁₁, wherein X₁ is G, E, T or A, X₂ is S, N, P, A, H, D, K, I or F, X₃ is I, S, V, T, D, L, A, M or H, X₄ is F, S, I, A, L or G, X₅ is N, S, G, A, D, T, E, R or H, X₆ is I, L, F, Y, E or N, X₇ is N, A, G, D, K, Y, L or S, X₈ is A, Y, V, D or N, X₉ is E or null, X₁₀ is F or null, X₁₁ is A or null.

In one or more embodiments, SEQ ID NO: 1 is X₁X₂X₃X₄X₅X₆X₇X₈, wherein X₁ is G, E, T or A, X₂ is S, N, P, A, H, D, K, I or F, X₃ is I, S, V, T, D, L, A, M or H, X₄ is F, S, I, A, L or G, X₅ is N, S, G, A, D, T, E or H, X₆ is I, L, F, Y or N, X₇ is N, A, G, D, K, Yor S, X₈ is A, Y, VorN.

In one or more embodiments, SEQ ID NO: 1 is GX₂X₃X₄XₛX₆X₇A, wherein X₂ is S, N, A, D, I or F, X₃ is I, V, T, D, A, L or S, X₄ is F, S, I, A or L, X₅ is N, S, A, D, E, T or H, X₆ is I, F, N or Y, X₇ is N, G, D or Y.

Preferably, SEQ ID NO: 1 is X₁X₂X₃X₄X₅X₆X₇X₈, wherein X₁ is G, E, T or A, X₂ is S, N, P, H or I, X₃ is I, S, T, D, L or A, X₄ is F, I or L, X₅ is S, T or E, X₆ is I, F or Y, X₇ is N, A, D or Y, X₈ is A or Y.

In one or more embodiments, CDR1 comprises the sequence shown in any one of SEQ ID NOs: 4-27, 73. Preferably, CDR1 comprises the sequence shown in any one of SEQ ID NOs: 5, 11, 14, 15, 17, 21, 27, 73.

In one or more embodiments, SEQ ID NO: 2 is X₁X₂X₃X₄X₅X₆X₇XₛX₉X₁₀X₁₁X₁₂, wherein X₁ is I, M, A, T or L, X₂ is S, G, N, D, T or V, X₃ is S, N, A, R, G or T, X₄ is S, T, G, D or N, X₅ is N, G, T or I, X₆ is S, R, N, D, K, T or G, X₇ is D, T, S, I or K, X₈ is N, T, G, D or null, X₉ is T, K, G or null, X₁₀ is G, R or null, X₁₁ is V, T or null, X₁₂ is T or null. In one or more embodiments, X₃ is S, N, A, R or T.

In one or more embodiments, SEQ ID NO: 2 is IX₂X₃X₄X₅X₆X₇X₈X₉, wherein X₂ is S, G, N or D, X₃ is S, N, A or T, X₄ is S, T, G, D or N, X₅ is Nor G, X₆ is S, R, N, D or K, X₇ is D, T, S or K, X₈ is N, T or null, X₉ is T, K or null.

Preferably, SEQ ID NO: 2 is X₁X₂X₃X₄X₅X₆X₇XₛX₉X₁₀X₁₁X₁₂, wherein X₁ is I, A or T, X₂ is S, G, N or T, X₃ is S or R, X₄ is T, G or D, X₅ is N, G or T, X₆ is S, R, N, K or T, X₇ is D, T, S, I or K, X₈ is N, G or null, X₉ is T, G or null, X₁₀ is G or null, X₁₁ is V or null, X₁₂ is T or null.

In one or more embodiments, CDR2 comprises the sequence shown in any one of SEQ ID NOs: 28-47, 74. Preferably, CDR2 comprises the sequence shown in any one of SEQ ID NOs:29, 35, 37, 38, 39, 43, 40, 74.

In one or more embodiments, SEQ ID NO: 3 is X₁X₂X₃X₄X₅X₆X₇X₈X₉X₁₀X₁₁X₁₂X₁₃X₁₄X₁₅X₁₆X₁₇X₁₈X₁₉X₂₀X₂₁X₂₂X₂₃, wherein X₁ is N, H, A or Q, X₂ is L, A, G or V, X₃ is S, R, E, G, D or T, X₄ is N, A, R, G, D, K, V, T, I, E or Q, X₅ is Y, F, K, S, C, I, D, G, H or R, X₆ is D, A, V, G, R, N, P, T, C, Q or S, X₇ is R, Y, T, A, D, K, L, E, S or G, X₈ is K, S, F, T, D, Q, E, G, P, R, Y or N, X₉ is D, G, I, H, S, Y, Q, K, T, R or V, X₁₀ is R, Y, H, N, T, D, P, V, K, C, L or S, X₁₁ is Y, D, E, P, F, S, L, I or null, X₁₂ is P, V, Q, A, Y, F, N, D, R or null, X₁₃ is D, A, Y, V, Q, P or null, X₁₄ is Y, L, P, A, E or null, X₁₅ is C, S, M, P, T or null, X₁₅ is C, S, M, P or null, X₁₆ is V, D, S, Y or null, X₁₇ is L, F, V, D or null, X₁₈ is R, G, M, S or null, X₁₉ is D, N, S or null, X₂₀ is Y, L or null, X₂₁ is Y or null, X₂₂ is A or null, X₂₃ is D or null.

In one or more embodiments, SEQ ID NO: 3 is X₁X₂X₃X₄X₅X₆X₇X₈X₉X₁₀X₁₁X₁₂X₁₃X₁₄X₁₅X₁₆X₁₇X₁₈X₁₉X₂₀X₂₁X₂₂X₂₃, wherein X₁ is N, H, A or Q, X₂ is L, A, G or V, X₃ is S, R, E, G, D or T, X₄ is N, A, R, G, D, K, V, T, I or Q, X₅ is Y, F, K, S, C, I, D, G, H or R, X₆ is D, A, V, G, R, N, P, T, C, Q or S, X₇ is R, Y, T, A, D, K, L, E, S or G, X₈ is K, S, F, T, D, Q, E, G, P, R, Y or N, X₉ is D, G, I, H, S, Y, Q, K, T or V, X₁₀ is R, Y, H, N, T, D, P, V, K, C or S, X₁₁ is Y, D, E, P, F, S, L or null, X₁₂ is P, V, Q, A, Y, F, N, D or null, X₁₃ is D, A, Y, V, Q or null, X₁₄ is Y, L, P, A, E or null, X₁₅ is C, S, M, P or null, X₁₆ is V, D, S, Y or null, X₁₇ is L, F, V, D or null, X₁₈ is R, G, M, S or null, X₁₉ is D, N, S or null, X₂₀ is Y, L or null, X₂₁ is Y or null, X₂₂ is A or null, X₂₃ is D or null.

In one or more embodiments, SEQ ID NO: 3 is AX₂X₃X₄X₅X₆X₇X₈X₉X₁₀X₁₁X₁₂X₁₃X₁₄X₁₅X₁₆X₁₇X₁₈X₁₉, wherein X₂ is A, G or V, X₃ is S, E, G, D or T, X₄ is N, R, D, K, T or I, X₅ is Y, F, K, I, D, G, H or R, X₆ is D, A, G, N, P, T, C, Q or S, X₇ is R, Y, T, A, D, K, L or G, X₈ is K, S, F, D, Q, G, P, Y or N, X₉ is D, G, I, S, Y, Q, K or V, X₁₀ is R, Y, H, T, D, P, V, K or C, X₁₁ is Y, D, E, F, S, L or null, X₁₂ is P, V, A, Y, F, N, D or null, X₁₃ is D, A, Y, V or null, X₁₄ is Y, P, E or null, X₁₅ is S, P or null, X₁₆ is D, Y or null, X₁₇ is F, D or null, X₁₈ is G, S or null, X₁₉ is N or null.

Preferably, SEQ ID NO: 3 is AX₂X₃X₄X₅X₆X₇X₈X₉X₁₀X₁₁X₁₂X₁₃X₁₄X₁₅X₁₆X₁₇X₁₈X₁₉, wherein X₂ is A or V, X₃ is S, E, G or D, X₄ is N, R, D or I, X₅ is Y, F, K, D or H, X₆ is D, G, P, C or S, X₇ is R, Y, D, K or L, X₈ is K, Q, G or N, X₉ is D, S, Y or Q, X₁₀ is R, Y, D, V or S, X₁₁ is Y, D, F, S, L or null, X₁₂ is P, F or null, X₁₃ is D, V or null, X₁₄ is P or null, X₁₅ is S or null, X₁₆ is D or null, X₁₇ is F or null, X₁₈ is G or null, X₁₉ is N or null.

In one or more embodiments, the anti-mesothelin single domain antibody is a specific single domain antibody to the segment III of mesothelin (the proximal membrane end).

In one or more embodiments, CDR3 comprises the sequence shown in any one of SEQ ID NOs: 48-72, 75. Preferably, CDR3 comprises the sequence shown in any one of SEQ ID NOs:49, 55, 58, 59, 61, 65, 72, 75.

In one or more embodiments, CDR1 of the single domain antibody comprises the sequence shown in any one of SEQ ID NOs: 4-27 and 73, CDR2 comprises the sequence shown in any one of SEQ ID NOs: 28-47 and 74, and CDR3 comprises the sequence shown in any one of SEQ ID NOs:48-72 and 75.

In one or more embodiments, the single domain antibody comprises CDR1, CDR2, and CDR3 shown in any one of the following groups a1 to a26:

| Group | CDR1 | CDR2 | CDR3 |
|---|---|---|---|
| a1 | 4 | 28 | 48 |
| a2 | 5 | 29 | 49 |
| a3 | 6 | 30 | 50 |
| a4 | 7 | 31 | 51 |
| a5 | 8 | 32 | 52 |
| a6 | 9 | 33 | 53 |
| a7 | 10 | 34 | 54 |
| a8 | 11 | 35 | 55 |
| a9 | 12 | 36 | 56 |
| a10 | 13 | 32 | 57 |
| a11 | 14 | 37 | 58 |
| a12 | 15 | 38 | 59 |
| a13 | 16 | 31 | 60 |
| a14 | 17 | 39 | 61 |
| a15 | 18 | 40 | 62 |
| a16 | 19 | 41 | 63 |
| a17 | 20 | 42 | 64 |
| a18 | 21 | 43 | 65 |
| a19 | 22 | 32 | 66 |
| a20 | 13 | 32 | 67 |
| a21 | 23 | 44 | 68 |
| a22 | 24 | 45 | 69 |
| a23 | 25 | 46 | 70 |
| a24 | 26 | 47 | 71 |
| a25 | 27 | 40 | 72 |
| a26 | 73 | 74 | 75 |

In one or more embodiments, the FR1 of the single domain antibody VHH can be selected from the FR1 of VHH of each antibody numbering in Table 1, the FR2 of VHH can be selected from the FR2 of VHH of each antibody numbering in Table 1, the FR3 of VHH can be selected from the FR3 of VHH of each antibody numbering in table 1, and the FR4 of VHH can be selected from the FR4 of VHH of each antibody numbering in Table 1.

In one or more embodiments, the FR region of the single domain antibody is the FR region of any VHH selected from SEQ ID NOs: 76-120.

In one or more embodiments, the single domain antibody VHH is as shown in any one of SEQ ID NOs: 76-120. Preferably, the single domain antibody is as shown in any one of SEQ ID NOs: 74, 83, 86, 87, 89, 93, 100, 103.

In one or more embodiments, the mesothelin binding molecule is a monovalent or multivalent single domain antibody, a multispecific single domain antibody, a heavy chain antibody or an antigen binding fragment thereof, an antibody or an antigen binding fragment thereof comprising one, two or more of the anti-mesothelin single domain antibodies described herein.

In one or more embodiments, the multivalent single domain antibody or multispecific single domain antibody connects a plurality of single domain antibodies through a linker. The linker consists of 1-15 amino acids selected from G and S.

In one or more embodiments, the antigen binding fragment of the heavy chain antibody is a single chain heavy chain antibody.

In one or more embodiments, the heavy chain antibody is a camelid heavy chain antibody or a shark heavy chain antibody.

In one or more embodiments, the heavy chain antibody further comprises a heavy chain constant region.

In one or more embodiments, the heavy chain constant region is a constant region of camelid heavy chain antibody, comprising CH2 and CH3. In one or more embodiments, the CH2 and CH3 are CH2 and CH3 of human IgG Fc, such as CH2 and CH3 of IgG4. Preferably, the heavy chain constant region is shown in SEQ ID NO: 121.

In one or more embodiments, the heavy chain constant region is a constant region of the shark heavy chain antibody, comprising CH1, CH2, CH3, CH4, and CH5.

In one or more embodiments, the antibody is an antibody comprising the anti-mesothelin single domain antibody as the heavy chain variable domain.

In one or more embodiments, the antibody further comprises a light chain variable domain, a heavy chain constant domain, and a light chain constant domain.

In one or more embodiments, the antigen binding fragment of the antibody is selected from Fab, F(ab')2, Fv, scFv.

In one or more embodiments, the binding molecule described in any embodiment of the present description is a chimeric antibody or a fully human antibody; preferably, a fully human antibody.

The description also provides a polynucleotide, comprising a sequence selected from:
(1) the coding sequence of the single domain antibody or the antibody or the antigen binding fragment thereof according to any embodiment herein;
(2) the complementary sequence of (1);
(3) a 5-50bp fragment of any sequence of (1) or (2).

In one or more embodiments, the fragment is a primer.

The description also provides a nucleic acid construct comprising the polynucleotide described herein.

In one or more embodiments, the nucleic acid construct is a recombinant vector or expression vector.

The description also provides a phage comprising the mesothelin binding molecule according to any embodiment herein.

In one or more embodiments, the mesothelin binding molecule is displayed on the surface of the phage.

The description also provides a host cell selected from:
(1) the host cell expressing the mesothelin binding molecule according to any embodiment herein;
(2) the host cell comprising a polynucleotide described herein; and/or
(3) the host cell comprising a nucleic acid construct described herein.

The description also provides a method for producing a mesothelin binding molecule, comprising culturing the host cells described herein under conditions suitable for producing the mesothelin binding molecule (such as monovalent or multivalent single domain antibodies, multispecific single domain antibodies, heavy chain antibodies, antibodies or antigen binding fragments thereof), and optionally purifying the mesothelin binding molecule from culture.

The description also provides a pharmaceutical composition, comprising the mesothelin binding molecule, polynucleotide, nucleic acid construct, phage or host cell according to any embodiment herein, and a pharmaceutically acceptable excipient.

In one or more embodiments, the pharmaceutical composition is used for treating cancer.

In one or more embodiments, the cancer is mesothelin related cancer. Preferably, the cancer includes mesothelioma, pancreatic cancer, ovarian cancer, lung adenocarcinoma, gastric cancer, etc.

The description also provides use of the mesothelin binding molecule according to any embodiment herein in the preparation of a medicament for the prevention or treatment of cancer.

In one or more embodiments, the cancer is mesothelin related cancer. Preferably, the cancer includes mesothelioma, pancreatic cancer, ovarian cancer, lung adenocarcinoma, gastric cancer, etc.

The description also provides a method for treating or preventing cancer, comprising administrating a patient in need thereof an effective amount of a mesothelin binding molecule according to any embodiment of the description, or a pharmaceutical compisiton comprising a mesothelin binding molecule according to any embodiment of the description.

In one or more embodiments, the cancer is a mesothelin related cancer. Preferably, the cancer includes mesothelioma, pancreatic cancer, ovarian cancer, lung adenocarcinoma, gastric cancer, etc.

The description also provides a kit for detecting mesothelin, for use in evaluating the therapeutic effect of a medicament or diagnosing cancer. The kit includes a mesothelin binding molecule, polynucleotide, nucleic acid construct, phage, and host cell according to any embodiment of the description.

In one or more embodiments, the kit further includes a reagent for detecting the binding of mesothelin to a single domain antibody, an antibody, or an antigen binding fragment thereof. For example, the bound reagent is detected by the enzyme-linked immunosorbent assay.

In one or more embodiments, the detection reagent for binding is a detectable marker, such as biotin, that can be linked to a mesothelin binding molecule. The detectable marker is connected to the mesothelin binding molecule or present in the kit separately.

The description also provides a non diagnostic method for detecting the presence of mesothelin in a sample. The method comprises: incubating a mesothelin binding molecule according to any embodiment herein with the sample, and detecting the binding of mesothelin to a single domain antibody, antibody, or antigen binding fragment thereof, thereby determining the presence of mesothelin in the sample. The detection is an enzyme-linked immunosorbent assay.

The description also provides use of a mesothelin binding molecule according to any embodiment herein in the preparation of a kit for detecting mesothelin in a sample, evaluating the therapeutic effect of a medicament or diagnosing a cancer.

### Description of the drawings

Figure 1 shows the detection of titer of Alpaca antiserum against MSLN protein.
Figure 2 shows the detection of titer of Alpaca antiserum against MSLN overexpressing cell line.
Figure 3 shows the detection of binding of candidate antibodies to MSLN segment III protein overexpressing cell line.
Figure 4 shows the detection of binding of candidate antibodies to SKOV3 tumor cell line.
Figure 5 shows the detection of binding of candidate antibodies to Aspc-1 tumor cell line.
Figure 6A and 6B show the detection of epitope competition of M044 antibody and YP218 antibody, wherein Figure 6A shows the detection of M044 antibody binding after YP218 antibody binding, and the binding order of Figure 6B is opposite to that of Figure 6A.

EC50 is present in nM. Isotype refers to an isotype control (negative control), and its amino acid sequence is shown in SEQ ID NO: 2 in CN106046152A.

### Detailed description

After extensive and in-depth research and a large number of screening, the inventor finds a class of mesothelin binding molecules comprising anti-mesothelin single domain antibodies. The experimental results show that the binding molecules of the description could specifically recognize the proximal membrane end of mesothelin, bind to mesothelin or mesothelin expressing cells and tumor cells with high affinity, and have no tissue cross-reactivity. The single domain antibody of the description is simple to generate.

Specifically, the invention immunizes Alpaca with human mesothelin protein to obtain a high-quality immune single domain antibody gene library. Then the MSLN protein and the proximal membrane fragment are coupled on a microtiter plate, and the immune single domain antibody gene library is screened by phage display technology, so as to obtain the MSLN specific single domain antibody gene. Then the gene was transferred to mammalian cells, and a single domain antibody strain with high specificity and high expression in mammalian cells is obtained. Then single domain antibodies with high avidity and low tissue cross-reactivity are identified by plasma resonance technology, flow cytometry, epitope competition test, biotin reporter gene detection system and other methods.

### Antibody

"Mesothelin binding molecule" or "MSLN binding molecule" as used herein is a protein that specifically binds mesothelin, including but not limited to antibodies, antigen binding fragments of antibodies, heavy chain antibodies, nano antibodies, micro antibodies, affibodies, target binding regions of receptors, cell adhesion molecules, ligands, enzymes, cytokines, and chemokines. "MD50" described herein is a protein with 94 amino acids long proximal to the membrane of mesothelin (GenBank: AAH09272.1, 487-580).

Term "antibody" as used herein includes monoclonal antibodies (including full-length antibodies with immunoglobulin Fc region), antibody compositions with polyepitopic specificity, multi-specific antibodies (e.g., bispecific antibodies), diabodies and single chain molecules, and antibody fragments, especially antigen binding fragments, (e.g., Fab, F(ab')2, and FV). The term "immunoglobulin" (Ig) is used interchangeably with "antibody" herein.

The basic 4-chain antibody unit is a heterotetrameric glycoprotein composed of two identical light chains (L) and two identical heavy chains (H). IgM antibody consists of 5 basic heterotetramer units and another polypeptide called J chain, which contains 10 antigen binding sites; IgA antibody contains 2-5 basic 4 chain units, which can polymerize with J chain to form a multivalent assemblages. In the case of IgGs, the 4-chain unit is typically about 150,000 daltons. Each light chain is connected to a heavy chain through a covalent disulfide bond, while the two heavy chains are connected to each other through one or more disulfide bonds, and the number of disulfide bonds depends on the isotype of the heavy chain. Each heavy and light chain also has an interchain disulfide bridge with regular spacing. Each heavy chain has a variable domain (VH) at the N-terminus, followed by three constant domains (for each α and γ chain, CH1, CH2, and CH3) and four constant domains (for µ and ε isoforms, CH1, CH2, CH3, and CH4) and the hinge region (Hinge) between the CH1 domain and the CH2 domain. Each light chain has a variable domain (VL) at the N-terminus, followed by a constant domain (CL) at the other end. VL is aligned with VH, while CL is aligned with the first constant domain (CH1) of the heavy chain. Specific amino acid residues are thought to form an interface between light and heavy chain variable domains. The paired VH and VL together form an antigen binding site. For the structures and properties of different classes of antibodies, see, for example, Basic and Clinical Immunology, 8th Edition, Daniel P. Sties, Abba I. Terr, and Tristram G. Parsolw, ed, Appleton & Lange, Norwalk, CT, 1994, page 71, and Chapter 6. The L chain from any vertebrate species can be assigned to one of two clearly distinct types, called kappa and lambda, based on the amino acid sequences of their constant domains.. Depending on the amino acid sequence of the constant domain of their heavy chains (CH), immunoglobulins can be assigned to different classes or isotypes. There are five classes of immunoglobulins: IgA, IgD, IgE, IgG and IgM, having heavy chains designated α, δ, ε, γ and µ, respectively. The γ and α classes are further divided into subclasses on the basis of relatively minor differences in the CH sequence and function, e.g., humans express the following subclasses: IgGl, IgG2A, IgG2B, IgG3, IgG4, IgA1 and IgA2.

"Heavy chain antibody" described herein is an antibody derived from camelidae or sharks. Compared with the above 4-chain antibody, the heavy chain antibody lacks light chain and heavy chain constant region 1 (CH1), and only contains two heavy chains composed of variable region (VHH) and other constant regions, wherein the variable region is connected to the constant region through a hinge region like structure. Each heavy chain of camelid heavy chain antibody contains one variable region (VHH) and two constant regions (CH2 and CH3), and each heavy chain of shark heavy chain antibody contains one variable region and five constant regions (CH1-CH5). Antigen binding fragments of heavy chain antibodies include VHH and single chain heavy chain antibodies. Heavy chain antibodies may have CH2 and CH3 of human IgG Fc by fusing with the constant region of human IgG Fc.

As used herein, the terms "single domain antibody", "anti-mesothelin single domain antibody", "heavy chain variable region domain of heavy chain antibody", "VHH" and "nanobody" can be used interchangeably, and all refer to single domain antibodies that specifically recognize and bind to mesothelin. Single domain antibodies are variable regions of heavy chain antibodies. Typically, single domain antibodies contain three CDRs and four FRs. Preferably, the single domain antibody of the description has CDR1 shown in SEQ ID NO: 1, CDR2 shown in SEQ ID NO: 2, and CDR3 shown in SEQ ID NO: 3. Single domain antibodies are the smallest functional antigen binding fragments. Generally, after obtaining an antibody which naturally lacks light chain and heavy chain constant region 1 (CH1), the variable region of the heavy chain of the antibody is cloned to construct a single domain antibody consisting of only one heavy chain variable region.

Binding molecules containing two or more single domain antibodies are multivalent single domain antibodies; and binding molecules containing two or more single domain antibodies with different specificities are multispecific single domain antibodies. Multivalent single domain antibodies or multispecific single domain antibodies are connected to multiple single domain antibodies through linkers. The linker usually consists of 1-15 amino acids selected from G and S.

The terms "heavy chain antibody" and "antibody" herein are intended to distinguish different composition forms of antibodies. Due to the similarity of their structures, the following descriptions on structures of antibodies except for light chains also apply to heavy chain antibodies.

The "variable region" or "variable domain" of an antibody refers to the amino-terminal domains of the heavy or light chain of the antibody. The variable domains of the heavy chain and light chain may be referred to as "VH" and "VL", respectively. These domains are generally the most variable parts of the antibody (relative to other antibodies of the same class) and contain the antigen binding sites.

The term "variable" refers to the fact that certain segments of the variable domains differ extensively in sequence among antibodies. The V domain mediates antigen binding and defines the specificity of a particular antibody for its particular antigen. However, the variability is not evenly distributed across the entire span of the variable domains. Instead, it is concentrated in three segments called hypervariable regions (HVRs) both in the light-chain and the heavy chain variable domains, namely HCDR1, HCDR2 and HCDR3 of heavy chain variable region (CDR1, CDR2 and CDR3 in heavy chain antibodies for short) and LCDR1, LCDR2 and LCDR3 of light chain variable region. The more highly conserved portions of variable domains are called the framework regions (FR). The variable domains of native heavy and light chains each comprise four FR regions (FR1, FR2, FR3, and FR4), largely adopting a beta-sheet configuration, connected by three HVRs, which form loops connecting, and in some cases forming part of, the beta-sheet structure. The HVRs in each chain are held together in close proximity by the FR regions and, with the HVRs from the other chain, contribute to the formation of the antigen binding site of antibodies. Generally, the structure of the light chain variable region is FR1-LCDR1-FR2-LCDR2-FR3-LCDR3-FR4, and the structure of the heavy chain variable region is FR1-HCDR1-FR2-HCDR2-FR3-HCDR3-FR4. The constant domains are not involved directly in the binding of antibody to an antigen, but exhibit various effector functions, such as participation of the antibody in antibody-dependent cellular toxicity.

An "Fc region" (fragment crystallizable region) or "Fc domain" or "Fc" refers to the C-terminal region of the heavy chain of an antibody that mediates the binding of the immunoglobulin to host tissues or factors, including binding to Fc receptors located on various cells of the immune system (e.g., effector cells) or to the first component (Clq) of the classical complement system. In IgG, IgA and IgD antibody isotypes, the Fc region is composed of two identical protein fragments, derived from CH2 and CH3 constant domains of the antibody's two heavy chains; IgM and IgE Fc regions contain three heavy chain constant domains (CH domains 2-4) in each polypeptide chain. Although the boundaries of the Fc region of an immunoglobulin heavy chain might vary, the human IgG heavy chain Fc region is usually defined to stretch from an amino acid residue at position C226 or P230 to the carboxy-terminus of the heavy chain, wherein the numbering is according to the EU index as in Kabat. As used herein, the Fc region may be a native sequence Fc or a variant Fc.

An "antibody fragment" comprises a portion of an intact antibody, preferably the antigen binding and/or the variable region of the intact antibody. The antibody fragment is preferably an antigen binding fragment of the antibody. Examples of antibody fragments include Fab, Fab', F(ab')2 and Fv fragments; diabodies; linear antibodies; single-chain antibody molecules, scFv-Fc fragment; multispecific antibodies formed from antibody fragments; and any fragment that should be able to increase the half-life by chemical modification or by incorporation into liposomes. Papain digestion of antibodies produced two identical antigen-binding fragments, called "Fab" fragments, and a residual "Fc" fragment, a designation reflecting the ability to crystallize readily. The Fab fragment consists of an entire L chain along with the variable region domain of the H chain (VH), and the first constant domain of one heavy chain (CH1). Each Fab fragment is monovalent with respect to antigen binding, i.e., it has a single antigen-binding site. Pepsin treatment of an antibody yields a single large F(ab')2 fragment which roughly corresponds to two disulfide linked Fab fragments having different antigen-binding activity and is still capable of cross-linking antigen. Fab' fragments differ from Fab fragments by having a few additional residues at the carboxy terminus of the CH1 domain including one or more cysteines from the antibody hinge region. F(ab')2 antibody fragments originally were produced as pairs of Fab' fragments which have hinge cysteines between them. Other chemical couplings of antibody fragments are also known. The Fc fragment comprises the carboxy-terminal portions of both H chains held together by disulfides. The effector functions of antibodies are determined by sequences in the Fc region, the region which is also recognized by Fc receptors (FcR) found on certain types of cells.

"Fv" is the minimum antibody fragment which contains a complete antigen-recognition and -binding site. This fragment consists of a dimer of one heavy- and one light-chain variable region domain in tight, non-covalent association. From the folding of these two domains emanate six hypervariable loops (3 loops each from the H and L chain) that contribute the amino acid residues for antigen binding and confer antigen binding specificity to the antibody. However, even a single variable domain (or half of an Fv comprising only three HVRs specific for an antigen) has the ability to recognize and bind antigen, although at a lower affinity than the entire binding site. "Single-chain Fv " also abbreviated as "sFv " or "scFv " are antibody fragments that comprise the VH and VL antibody domains connected into a single polypeptide chain. Preferably, the sFv polypeptide further comprises a polypeptide linker between the VH and VL domains which enables the sFv to form the desired structure for antigen binding.

The term "monoclonal antibody" as used herein refers to an antibody obtained from a population of substantially homogeneous antibodies, i.e., the individual antibodies comprising the population are identical except for possible naturally occurring mutations and/or post-translation modifications (e.g., isomerizations, amidations) that may be present in minor amounts. Monoclonal antibodies are highly specific, being directed against a single antigenic site. In contrast to polyclonal antibody preparations which typically include different antibodies directed against different determinants (epitopes), each monoclonal antibody is directed against a single determinant on the antigen. In addition to their specificity, the monoclonal antibodies are advantageous in that they are synthesized by the hybridoma culture, uncontaminated by other immunoglobulins. The modifier "monoclonal" indicates the character of the antibody as being obtained from a substantially homogeneous population of antibodies, and is not to be construed as requiring production of the antibody by any particular method. For example, the monoclonal antibodies to be used in accordance with the present invention may be made by a variety of techniques, including, for example, the hybridoma method, phage-display technologies, recombinant DNA methods, and technologies for producing human or humanlike antibodies in animals that have parts or all of the human immunoglobulin loci or genes encoding human immunoglobulin sequences, single-cell sequencing methods.

The monoclonal antibodies herein specifically include "chimeric" antibodies in which a portion of the heavy and/or light chain is identical with or homologous to corresponding sequences in antibodies derived from a particular species or belonging to a particular antibody class or subclass, while the remainder of the chain(s) is(are) identical with or homologous to corresponding sequences in antibodies derived from another species or belonging to another antibody class or subclass, as well as fragments of such antibodies, so long as they exhibit the desired biological activity.

"Humanized" forms of non-human (e.g., murine) antibodies are chimeric antibodies that contain minimal sequence derived from non-human immunoglobulin. Therefore, "humanized antibodies" generally refer to non-human antibodies that have had the variable- domain framework regions swapped for sequences found in human antibodies. Generally, in a humanized antibody, the entire antibody, except the CDRs, is encoded by a polynucleotide of human origin or is identical to such an antibody except within its CDRs. The CDRs, some or all of which are encoded by nucleic acids originating in a non-human organism, are grafted into the beta-sheet framework of a human antibody variable region to create an antibody, the specificity of which is determined by the engrafted CDRs. The creation of such antibodies are well known in the art, such as using mice with genetically engineered immune systems. In the description, antibodies, single domain antibodies, heavy chain antibodies, etc. all include humanized variants of the antibodies.

A "human antibody" is an antibody that possesses an amino-acid sequence corresponding to that of an antibody produced by a human and/or has been made using any of the techniques for making human antibodies as disclosed herein. This definition of a human antibody specifically excludes a humanized antibody comprising non-human antigen-binding residues. Human antibodies can be produced using various techniques known in the art, including phage-display libraries.

In some embodiments, the description also provides a single domain antibody, heavy chain antibody, antibody or antigen binding fragment thereof that binds to the same epitope of mesothelin (such as the proximal membrane end of mesothelin III segment or MD50) as any anti-mesothelin single domain antibody of the description, that is, a single domain antibody, heavy chain antibody, antibody or antigen binding fragment thereof that can cross-compete with any single domain antibody of the description for binding to mesothelin.

In this description, CDR1 of the single domain antibody comprises the sequence shown in SEQ ID NO: 1, which is X₁X₂X₃X₄X₅X₆X₇X₈X₉X₁₀X₁₁, wherein X₁ is G, E, T or A, X₂ is S, N, P, A, H, D, K, I or F, X₃ is I, S, V, T, D, L, A, M or H, X₄ is F, S, I, A, L or G, X₅ is N, S, G, A, D, T, E, R or H, X₆ is I, L, F, Y, E or N, X₇ is N, A, G, D, K, Y, L or S, X₈ is A, Y, V, D or N, X₉ is E or null, X₁₀ is F or null, X₁₁ is A or null. In one or more embodiments, SEQ ID NO: 1 is X₁X₂X₃X₄X₅X₆X₇X₈, wherein X₁ is G, E, T or A, X₂ is S, N, P, A, H, D, K, I or F, X₃ is I, S, V, T, D, L, A, M or H, X₄ is F, S, I, A, L or G, X₅ is N, S, G, A, D, T, E orH, X₆ is I, L, F, Y orN, X₇ is N, A, G, D, K, Yor S, X₈ is A, Y, VorN. Preferably, X₁ is G, E, T or A, X₂ is S, N, P, H or I, X₃ is I, S, T, D, L or A, X₄ is F, I or L, X₅ is S, T or E, X₆ is I, F or Y, X₇ is N, A, D or Y, X₈ is A or Y. In one or more embodiments, SEQ ID NO: 1 is GX₂X₃X₄X₅X₆X₇A, wherein X₂ is S, N, A, D, I or F, X₃ is I, V, T, D, A, L or S, X₄ is F, S, I, A or L, X₅ is N, S, A, D, E, T or H, X₆ is I, F, N or Y, X₇ is N, G, D or Y. In one or more embodiments, CDR1 comprises the sequence shown in any one of SEQ ID NOs: 4-27, 73.

In the description, CDR2 of the single domain antibody comprises the sequence shown in SEQ ID NO: 2, which is X₁X₂X₃X₄X₅X₆X₇XₛX₉X₁₀X₁₁X₁₂, wherein X₁ is I, M, A, T or L, X₂ is S, G, N, D, T or V, X₃ is S, N, A, R, G or T, X₄ is S, T, G, D or N, X₅ is N, G, T or I, X₆ is S, R, N, D, K, T or G, X₇ is D, T, S, I or K, X₈ is N, T, G, D or null, X₉ is T, K, G or null, X₁₀ is G, R or null, X₁₁ is V, T or null, X₁₂ is T or null. Preferably, X₁ is I, A or T, X₂ is S, G, N or T, X₃ is S or R, X₄ is T, G or D, X₅ is N, G or T, X₆ is S, R, N, K or T, X₇ is D, T, S, I or K, X₈ is N, G or null, X₉ is T, G or null, X₁₀ is G or null, X₁₁ is V or null, X₁₂ is T or null. In one or more embodiments, X₃ is S, N, A, R, or T. In one or more embodiments, SEQ ID NO: 2 is IX₂X₃X₄X₅X₆X₇X₈X₉, wherein X₂ is S, G, N or D, X₃ is S, N, A or T, X₄ is S, T, G, D or N, X₅ is N or G, X₆ is S, R, N, D or K, X₇ is D, T, S or K, X₈ is N, T or null, X₉ is T, K or null. In one or more embodiments, CDR2 comprises the sequence shown in any one of SEQ ID NOs: 28-47, 74.

In the description, CDR3 of the single domain antibody comprises the sequence shown in SEQ ID NO: 3, which is X₁X₂X₃X₄X₅X₆X₇X₈X₉X₁₀X₁₁X₁₂X₁₃X₁₄X₁₅X₁₆X₁₇X₁₈X₁₉X₂₀X₂₁X₂₂X₂₃, wherein X₁ is N, H, A or Q, X₂ is L, A, G or V, X₃ is S, R, E, G, D or T, X₄ is N, A, R, G, D, K, V, T, I, E or Q, X₅ is Y, F, K, S, C, I, D, G, H or R, X₆ is D, A, V, G, R, N, P, T, C, Q or S, X₇ is R, Y, T, A, D, K, L, E, S or G, X₈ is K, S, F, T, D, Q, E, G, P, R, Y or N, X₉ is D, G, I, H, S, Y, Q, K, T, R or V, X₁₀ is R, Y, H, N, T, D, P, V, K, C, L or S, X₁₁ is Y, D, E, P, F, S, L, I or null, X₁₂ is P, V, Q, A, Y, F, N, D, R or null, X₁₃ is D, A, Y, V, Q, P or null, X₁₄ is Y, L, P, A, E or null, X₁₅ is C, S, M, P, T or null, X₁₅ is C, S, M, P or null, X₁₆ is V, D, S, Y or null, X₁₇ is L, F, V, D or null, X₁₈ is R, G, M, S or null, X₁₉ is D, N, S or null, X₂₀ is Y, L or null, X₂₁ is Y or null, X₂₂ is A or null, X₂₃ is D or null. In one or more embodiments, X₁ is N, H, A or Q, X₂ is L, A, G or V, X₃ is S, R, E, G, D or T, X₄ is N, A, R, G, D, K, V, T, I or Q, X₅ is Y, F, K, S, C, I, D, G, H or R, X₆ is D, A, V, G, R, N, P, T, C, Q or S, X₇ is R, Y, T, A, D, K, L, E, S or G, X₈ is K, S, F, T, D, Q, E, G, P, R, Y or N, X₉ is D, G, I, H, S, Y, Q, K, T or V, X₁₀ is R, Y, H, N, T, D, P, V, K, C or S, X₁₁ is Y, D, E, P, F, S, L or null, X₁₂ is P, V, Q, A, Y, F, N, D or null, X₁₃ is D, A, Y, V, Q or null, X₁₄ is Y, L, P, A, E or null, X₁₅ is C, S, M, P or null, X₁₆ is V, D, S, Y or null, X₁₇ is L, F, V, D or null, X₁₈ is R, G, M, S or null, X₁₉ is D, N, S or null, X₂₀ is Y, L or null, X₂₁ is Y or null, X₂₂ is A or null, X₂₃ is D or null. In one or more embodiments, SEQ ID NO: 3 is AX₂X₃X₄X₅X₆X₇X₈X₉X₁₀X₁₁X₁₂X₁₃X₁₄X₁₅X₁₆X₁₇X₁₈X₁₉, wherein X₂ is A, G or V, X₃ is S, E, G, D or T, X₄ is N, R, D, K, T or I, X₅ is Y, F, K, I, D, G, H or R, X₆ is D, A, G, N, P, T, C, Q or S, X₇ is R, Y, T, A, D, K, L or G, X₈ is K, S, F, D, Q, G, P, Y or N, X₉ is D, G, I, S, Y, Q, K or V, X₁₀ is R, Y, H, T, D, P, V, K or C, X₁₁ is Y, D, E, F, S, L or null, X₁₂ is P, V, A, Y, F, N, D or null, X₁₃ is D, A Y, V or null, X₁₄ is Y, P, E or null, X₁₅ is S, P or null, X₁₆ is D, Y or null, X₁₇ is F, D or null, X₁₈ is G, S or null, X₁₉ is N or null. Preferably, X₁ is A, X₂ is A or V, X₃ is S, E, G or D, X₄ is N, R, D or I, X₅ is Y, F, K, D or H, X₆ is D, G, P, C or S, X₇ is R, Y, D, K or L, X₈ is K, Q, G or N, X₉ is D, S, Y or Q, X₁₀ is R, Y, D, V or S, X₁₁ is Y, D, F, S, L or null, X₁₂ is P, F or null, X₁₃ is D, V or null, X₁₄ is P or null, X₁₅ is S or null, X₁₆ is D or null, X₁₇ is F or null, X₁₈ is G or null, X₁₉ is N or null, X₂₀-X₂₃ is none. In one or more embodiments, CDR3 comprises the sequence shown in any one of SEQ ID NOs: 48-72, 75.

In one or more embodiments, CDR1 of the single domain antibody contains the sequence shown in any one of SEQ ID NOs: 5, 11, 14, 15, 17, 21, 27, 73, CDR2 contains the sequence shown in any one of SEQ ID NOs: 29, 35, 37, 38, 39, 43, 40, 74, and CDR3 contains the sequence shown in any one of SEQ ID NOs: 49, 55, 58, 59, 61, 65, 72, 75.

In one or more embodiments, the single domain antibody contains CDR1, CDR2, and CDR3 shown in any of groups a1 to a26 in Table 1:

**Table 1**

| Group | CDR1 | CDR2 | CDR3 | Antibody No. | VHH |
|---|---|---|---|---|---|
| a1 | 4 | 28 | 48 | M001 | 76 |
| a2 | 5 | 29 | 49 | M003 | 77 |
| a3 | 6 | 30 | 50 | M004 | 78 |
| a4 | 7 | 31 | 51 | M005 | 79 |
| a5 | 8 | 32 | 52 | M010 | 80 |
| a6 | 9 | 33 | 53 | M012 | 81 |
| a7 | 10 | 34 | 54 | M032 | 82 |
| a8 | 11 | 35 | 55 | M044 | 83 |
| a9 | 12 | 36 | 56 | M047 | 84 |
| a10 | 13 | 32 | 57 | M050 | 85 |
| a11 | 14 | 37 | 58 | M118 | 86 |
| a12 | 15 | 38 | 59 | M119 | 87 |
| a13 | 16 | 31 | 60 | M126 | 88 |
| a14 | 17 | 39 | 61 | M129 | 89 |
| a15 | 18 | 40 | 62 | M132 | 90 |
| a16 | 19 | 41 | 63 | M137 | 91 |
| a17 | 20 | 42 | 64 | M143 | 92 |
| a18 | 21 | 43 | 65 | M156 | 93 |
| a19 | 22 | 32 | 66 | M169 | 94 |
| a20 | 13 | 32 | 67 | M182 | 95 |
| a21 | 23 | 44 | 68 | M191 | 96 |
| a22 | 24 | 45 | 69 | M197 | 97 |
| a23 | 25 | 46 | 70 | M210 | 98 |
| a24 | 26 | 47 | 71 | M224 | 99 |
| a25 | 27 | 40 | 72 | M344 | 100 |
| a26 | 73 | 74 | 75 | M2339 etc | 101-120 |

It is preferred to contain CDR1, CDR2, and CDR3 selected from any of the following groups: a2, a8, a11, a12, a14, a18, a25, a26.

In one or more embodiments, FR1 of the single domain antibody VHH can be selected from the FR1 of VHH of each antibody numbering in Table 1, FR2 of VHH can be selected from the FR2 of VHH of each antibody numbering in Table 1, FR3 of VHH can be selected from the FR3 of VHH of each antibody numbering in Table 1, and FR4 of VHH can be selected from the FR4 of VHH of each antibody numbering in Table 1.

In the preferred embodiment, the FR region of the single domain antibody VHH of the description is the FR region of any VHH selected from SEQ ID NOs: 76-120. Further preferably, the CDRs of such antibodies are selected from any of the aforementioned groups a1 to a25. In one or more embodiments, the single domain antibody VHH is as shown in any one of SEQ ID NOs: 76-120. Preferably, the single domain antibody is as shown in any one of SEQ ID NOs: 77, 83, 86, 87, 89, 93, 100, 103.

The mesothelin binding molecule described herein may be a monovalent or multivalent single domain antibody, a multispecific single domain antibody, a heavy chain antibody, or an antigen binding fragment thereof, an antibody, or an antigen binding fragment thereof, comprising one, two, or more anti-mesothelin single domain antibodies described herein. The heavy chain antibody also comprises a heavy chain constant region, such as the constant region of camelid heavy chain antibody or shark heavy chain antibody. Preferably, the heavy chain constant region is shown in SEQ ID NO: 121.

The description also includes the antibody derivatives and analogues. "Derivatives" and "analogues" refer to polypeptides that basically maintain the same biological function or activity of the antibody of the present description. The derivatives or analogues of the present description may be polypeptides formed from (i) a polypeptide with a substituent group in one or more amino acid residues, or (ii) a polypeptide formed from fusion of a mature polypeptide with another compound, such as a compound that extends the half-life of the polypeptide, such as polyethylene glycol, or (iii) a polypeptide formed by fusing an additional amino acid sequence to this polypeptide sequence (such as a leader sequence or a secretory sequence, or a sequence or prokaryotic sequence used for purifying this polypeptide, or a fusion protein formed with a 6His tag). According to the teaching herein, these derivatives and analogues belong to common sense known to those skilled in the art.

Without substantially affecting the activity of the antibody, those skilled in the art may change one or more (for example, 1, 2, 3, 4, 5, 6, 7, 8, 9 or 10 or more) amino acids to the sequence of the description to obtain the variant of the antibody or the functional fragment sequence thereof. These variants include (but are not limited to): deletion, insertion, and/or substitution of one or more (usually 1-50, preferably 1-30, more preferably 1-20, and most preferably 1-10) amino acids, and addition of one or more (usually less than 20, preferably less than 10, and more preferably less than 5) amino acids at the C-terminus and/or N-terminus. In this field, conservative substitution with amino acids with similar or similar properties usually does not change the function of the protein. For example, substituting with amino acids having similar properties may be performed in the FR and/or CDR regions of the variable region. Amino acid residues available for conservative substitution are well known in the art. Such substituted amino acid residues may or may not be encoded by a genetic code. For another example, adding one or more amino acids to the C-terminus and/or N-terminus usually does not change the function of the protein. They are all considered to be included in the scope of the present description. Table 2 shows the VHH sequences of the M2339 antibody and its exemplary variants.

**Table 2**

| Antibody No. | Amino acid sequence of VHH |
|---|---|
| M2339 | SEQ ID NO:101 |
| M2339-z11-1 | SEQ ID NO:102 |
| M2339-z11-2 | SEQ ID NO:103 |
| M2339-z11-3 | SEQ ID NO:104 |
| M2339-z11-4 | SEQ ID NO:105 |
| M2339-z11-5 | SEQ ID NO:106 |
| M2339-z11-6 | SEQ ID NO:107 |
| M2339-z11-7 | SEQ ID NO:108 |
| M2339-z11-8 | SEQ ID NO:109 |
| M2339-z11-9 | SEQ ID NO:110 |
| M2339-z11-10 | SEQ ID NO:111 |
| M2339-z11-11 | SEQ ID NO:112 |
| M2339-z11-12 | SEQ ID NO:113 |
| M2339-z11-13 | SEQ ID NO:114 |
| M2339-z11-14 | SEQ ID NO:115 |
| M2339-z11-15 | SEQ ID NO:116 |
| M2339-z11-16 | SEQ ID NO:117 |
| M2339-z11-17 | SEQ ID NO:118 |
| M2339-z11-1 | SEQ ID NO:119 |
| M2339-z1-2 | SEQ ID NO:120 |

The variant forms of the antibody described herein include: homologous sequence, conservative variant, allelic variant, natural mutant, induced mutant, protein encoded by DNA that can hybridize with the coding DNA of the antibody of the description under high or low strictness conditions, and polypeptide or protein obtained by using the antiserum of the antibody of the description.

In some embodiments, the sequence of the variant of the present description may have at least 95%, 96%, 97%, 98% or 99% identity with its source sequence. The sequence identity described in the description can be measured using sequence analysis software. For example, the computer program BLAST with default parameters, especially BLASTP or TBLASTN. The description also includes those molecules with variable regions of antibody heavy chain with CDRs, if their CDRs have more than 90% homology (preferably more than 95%, more preeferably more than 98%) with the CDRs identified here.

The antibody of the description can be prepared by conventional methods in the art, such as hybridoma technology well known in the art. The heavy chain antibody of the description can be prepared by conventional methods in the art, such as phage display technology well known in the art. Alternatively, the antibodies or heavy chain antibodies of the present description may be expressed in other cell lines. Suitable mammalian host cells can be transformed with sequences encoding antibodies of the present description. Transformation can be carried out using any known method, including, for example, packaging polynucleotides in viruses (or viral vectors) and transducing host cells with the viruses (or vectors). The transformation procedure used depends on the host to be transformed. Methods for introducing heterologous polynucleotides into mammalian cells are well known in the art, including dextran mediated transfection, calcium phosphate precipitation, Polybrene mediated transfection, protoplast fusion, electroporation, encapsulation of polynucleotides in liposomes and direct microinjection of DNA into the nucleus. Mammalian cell lines that can be used as hosts for expression are well known in the art, including but not limited to a variety of immortalized cell lines available from the American Typical Culture Collection (ATCC), including but not limited to Chinese hamster ovary (CHO) cells, HeLa cells, baby hamster kidney (BHK) cells, monkey kidney cells (COS), human hepatocellular carcinoma cells (e.g., HepG2), etc. Particularly preferred cell lines are selected by determining which cell lines have high expression levels and produce antibodies with basic mesothelin binding properties.

### Nucleic acid

The description also provides polynucleotides encoding the above antibody or fragments thereof. Polynucleotides encoding heavy chain variable region, light chain variable region, heavy chain, light chain and CDRs are provided. The polynucleotide of the description can be in the form of DNAs or RNAs. DNAs include cDNAs, genomic DNAs, or synthetic DNAs. DNAs can be single stranded DNAs or double stranded DNAs. DNAs can be coding or noncoding strands.

As those skilled in the art will understand, due to the degeneracy of the genetic code, an extremely large number of nucleic acids can be prepared, all of which encode the antibody of the description or antigen binding fragment thereof. Therefore, when a specific amino acid sequence has been identified, those skilled in the art can simply modify the sequence of one or more codons without changing the amino acid sequence of the encoded protein to produce any number of different nucleic acids. Therefore, the present description also relates to polynucleotides that hybridize with the above polynucleotide sequences and have at least 50%, preferably at least 70%, more preferably at least 80% identity between the two sequences. The description particularly relates to polynucleotides that can hybridize with the polynucleotides of the description under strict conditions. In the present description, "strict conditions" refer to: (1) hybridization and elution at lower ionic strength and higher temperature, such as 0.2 × SSC, 0.1%SDS, 60 °C; or (2) addition of denaturants during hybridization, such as 50% (v/v) formamide, 0.1% calf serum/0.1% Ficoll, 42 °C, etc; or (3) hybridization that occurs only when the identity between two sequences is at least more than 90%, or preferably, more than 95%. Moreover, the polypeptides encoded by hybridizable polynucleotides have the same biological functions and activities as mature polypeptides.

The nucleotide full-length sequence of the antibody of the description or fragment thereof can usually be obtained by PCR amplification method, recombination method or artificial synthesis method. A feasible method is to synthesize relevant sequences by artificial synthesis, especially with short fragment length. Usually, fragments with very long sequence can be obtained by synthesizing several small fragments first and then connecting them. In addition, the coding sequence of the heavy chain and the expression tag (such as 6His) can also be fused together to form a fusion protein.

Once the relevant sequences are obtained, they can be obtained in large quantities by recombination. They are related sequences usually cloned into vectors, transferred into cells, and then isolated from the proliferated host cells by conventional methods. The biomolecules (nucleic acids, proteins, etc.) according to the present description include biomolecules in isolated form. At present, the DNA sequence encoding the protein (or fragment thereof, or derivative thereof) of the description can be obtained completely through chemical synthesis. This DNA sequence can then be introduced into various existing DNA molecules (or such as vectors) and cells known in the art. In addition, mutations can also be introduced into the protein sequence of the description through chemical synthesis.

Therefore, the present description also relates to nucleic acid constructs, such as expression vectors and recombinant vectors, containing the above appropriate DNA sequence and the appropriate promoter or control sequence. These vectors can be used to transform appropriate host cells to enable them to express proteins. Vectors usually contain sequences for plasmid maintenance and for cloning and expressing exogenous nucleotide sequences. The sequence (collectively referred to as "flanking sequence" in some embodiments) generally includes one or more of the following nucleotide sequences: promoter, one or more enhancer sequences, replication origin, transcription termination sequence, complete intronic sequence containing donor and receptor splice sites, sequence encoding leader sequence for polypeptide secretion, ribosome binding site, polyadenylation sequence, a multi-linker region for inserting nucleic acids encoding antibodies to be expressed and an optional marker element.

Host cells can be prokaryotic cells, such as bacterial cells; or lower eukaryotic cells, such as yeast cells; or higher eukaryotic cells, such as mammalian cells. Representative examples are: *Escherichia coli*, Streptomyces; bacterial cells of *Salmonella typhimurium*; fungal cells such as yeast; insect cells of Drosophila S2 or Sf9; animal cells of CHO, COS7, 293 cells, etc.

In some embodiments, host cells may be various functional cells well known in the art, such as various killer cells, including but not limited to cytokine induced killer cells (CIK), dendritic cell stimulated cytokine induced killer cells (DC-CIK), cytotoxic T lymphocytes (CTL), γδ T cells, natural killer cells (NK), tumor infiltrating lymphocytes (TIL), lymphokine activated killer cells (LAK), CD3AK cells (killer cells of anti-CD3 mAb), and CAR-T/TCR-T cells. In some embodiments, the killer cells are T cells or NK cells. Exemplary NK cells include, but are not limited to, primary NK cells, NK cell strains (such as NK92), and NKT cells. In some embodiments, the NK cells are primary NK cells. Exemplary T cells include, but are not limited to, peripheral blood T lymphocytes, cytotoxic T cells (CTLs), helper T cells, inhibitory/regulatory T cells, γδ T cells and mixed T cell populations of cytokine induced killer cells (CIK) and tumor infiltrating lymphocytes (TIL). In some embodiments, the T cells are peripheral blood T lymphocytes and TIL derived T cells.

Transformation of host cells with recombinant DNA can be performed using conventional techniques known to those skilled in the art. When the host is a prokaryote such as *Escherichia coli*, competent cells that can absorb DNA can be harvested after the exponential growth period and treated with CaCl₂ method, the steps of which are well known in the art. Another method involves the use of MgCl₂. If necessary, the transformation can also be performed by electroporation. When the host is eukaryote, the following DNA transfection methods can be selected: calcium phosphate coprecipitation method, conventional mechanical methods such as microinjection, electroporation, liposome packaging, etc.

The obtained transformants can be cultured by conventional methods to express the polypeptide encoded by the gene of the description. According to the host cells used, the medium used in the culture can be selected from various conventional media. Culture is performed under conditions suitable for host cell growth. When the host cells grow to the appropriate cell density, the selected promoters are induced by appropriate methods (such as temperature conversion or chemical induction), and the cells are cultured for another period of time.

The polypeptide in the above method can be expressed inside the cell, on the cell membrane, or secreted outside the cell. If necessary, the recombinant protein can be separated and purified by various separation methods using its physical, chemical, and other characteristics. These methods are familiar to those skilled in the art. Examples of these methods include but are not limited to: conventional renaturation treatment, treatment with protein precipitant (salting-out method), centrifugation, permeation, ultra-treatment, ultra-centrifugation, molecular sieve chromatography (gel filtration), adsorption chromatography, ion exchange chromatography, high-performance liquid chromatography (HPLC) and other various liquid chromatography technologies and combinations of these methods.

### Use of treatment and pharmaceutical composition

By constructing a nanoantibody library, the inventor found, expressed, and purified 25 nanoantibodies that could bind MSLN protein and MD50 protein. The binding of these antibodies to antigen and cells and drug safety were verified by affinity detection at protein level, affinity detection at cell level, MSLN binding detection for tumor cells, epitope competition experiments and tissue cross-reaction.

Therefore, all aspects of the antibodies described herein can be used to prepare medicaments to prevent or treat various conditions and diseases described herein, especially those related to mesothelin expressing cells. In some embodiments, the conditions and diseases are cancers, including but not limited to mesothelioma, pancreatic cancer, ovarian cancer, lung adenocarcinoma, gastric cancer, etc.

The pharmaceutical composition herein contains the binding molecules described herein, as well as a pharmaceutically acceptable excipient, including but not limited to diluents, vehicles, solubilizers, emulsifiers, preservatives, and/or adjuvants. The excipients are preferably non-toxic to the recipient at the dose and concentration used. Such excipients include (but are not limited to): saline, buffer, glucose, water, glycerol, ethanol, and their combinations. In some embodiments, the pharmaceutical composition may contain substances for improving, maintaining, or retaining, for example, the pH, permeability, viscosity, clarity, color, isotonicity, odor, sterility, stability, dissolution or release rate, absorption, or permeation of the composition. These substances are known in the prior art. The optimal pharmaceutical composition can be determined according to the expected route of administration, mode of delivery, and required dose.

Pharmaceutical compositions for *in vivo* administration are usually provided in the form of sterile formulation. Sterilization is achieved by filtration through a sterile filter membrane. When the composition is lyophilized, this method can be used for sterilization before or after lyophilization and rehydration. The pharmaceutical composition of the present description may be selected for parenteral delivery. Compositions for parenteral administration may be in lyophilized form or stored in solution. For example, it is prepared by conventional methods with normal saline or aqueous solution containing glucose and other adjuvants. Parenteral compositions are usually placed in containers with sterile access holes, such as intravenous solution strips or vials with plugs that can be pierced by subcutaneous injection needles. Alternatively, compositions may be selected for inhalation or delivery through the digestive tract, such as oral. The preparation of the pharmaceutically acceptable composition is within the art. Other pharmaceutical compositions will be apparent to those skilled in the art, including formulations containing antibodies in sustained or controlled release delivery formulations. The techniques used to prepare a variety of other sustained or controllable delivery modes (such as liposome carriers, bioerodible particles or porous beads, and deposit injection) are also known to those skilled in the art.

Once the pharmaceutical composition is prepared, it is stored in sterile vials in the form of solution, suspension, gel, emulsion, solid, crystal or dehydrated or lyophilized powder. The formulation may be stored in ready to use form or rehydrated before administration (e.g., lyophilized). The description also provides a kit for generating a single dose administration unit. The kit of the description can each contain a first container with dried protein and a second container with aqueous formulation. In some embodiments of the present description, kits containing single-chamber and multi-chamber pre-filled syringes (e.g., liquid syringes and lyophilized syringes) are provided.

The present description also provides a method for treating a patient (especially a patient's mesothelin related disease) by administering a binding molecule or a pharmaceutical composition thereof according to any embodiment of the present description. Terms "patient", "subject", "individual" and "object" are used interchangeably herein, including any organism, preferably animals, more preferably mammals (such as rats, mice, dogs, cats, rabbits, etc.), and most preferably humans. "Treatment" refers to the subject accepting the treatment scheme described herein to achieve at least one positive treatment effect (for example, a reduced number of cancer cells, a reduced tumor volume, a reduced rate of cancer cells infiltrating into peripheral organs, or a redeuced rate of tumor metastasis or tumor growth). The treatment scheme for effectively treating patients can vary according to many factors, such as the patient's disease status, age, weight, and the ability of the therapy to stimulate the subject's anti-cancer response.

The therapeutically effective amount of the pharmaceutical composition containing the binding molecule of the present description to be used will depend on, for example, the degree and the target of treatment. Those skilled in the art will understand that the appropriate dose level for treatment will vary in part depending on the molecule delivered, the indication, the route of administration, and the size (body weight, body surface or organ size) and/or condition (age and general health condition) of the patient. In some embodiments, clinicians may titrate the dose and change the route of administration to obtain the optimal therapeutic effect. For example, about 10 micrograms/kg body weight per day to about 50 mg/kg body weight.

The dosing frequency will depend on the pharmacokinetic parameters of the bound molecules in the formulation used. Clinicians typically administer the composition until a dose that achieves the desired effect. The composition may therefore be administered as a single dose, or over time as two or more doses (which may or may not contain the same amount of the desired molecule), or as a continuous infusion through an implanted device or catheter.

The route of administration of the pharmaceutical composition is according to known methods, such as oral, intravenous, intraperitoneal, intracerebral (intraparenchymal), intraventricular, intramuscular, intraocular, intra-arterial, portal vein or intralesional injection; by continuous release system or by implantable device.

### Diagnosis, detection, and kit

The binding molecule of the present description can be used in assays due to its high avidity with mesothelin, such as binding assays to detect and/or quantify mesothelin expressed in tissues or cells. Binding molecules such as single domain antibodies can be used in further studies investigating the function of mesothelin in disease. The methods for detecting mesothelin are roughly as follows: obtaining cell and/or tissue samples; and detecting the level of mesothelin in the sample.

The mesothelin binding molecule of the description can be used for diagnostic purposes to detect, diagnose, or monitor mesothelin related diseases and/or conditions. The description provides the detection of the presence of mesothelin in a sample using a classical immunohistological method known to those skilled in the art. Mesothelin can be detected *in vivo* or *in vitro.* Examples of methods suitable for detecting the presence of mesothelin include ELISA, FACS, RIA, etc.

For diagnostic uses, binding molecules such as single domain antibodies are usually labeled with detectable labeling groups. Suitable labeling groups include (but are not limited to): radioisotopes or radionuclides (e.g., 3H, 14C, 15N, 35S, 90Y, 99Tc, 1111n, 125I, 131I), fluorescent groups (e.g., FITC, rhodamine, lanthanide phosphors), enzymatic groups (e.g., horseradish peroxidase, β-galactosidase, luciferase, alkaline phosphatase), chemiluminescent groups, biotinyl groups, or predetermined polypeptide epitopes recognized by secondary reporters (e.g., leucine zipper pair sequences, binding sites for secondary antibodies, metal binding domains, epitope tags), MRI (magnetic resonance imaging) or CT (computed tomography) contrast agents. Various methods for labeling proteins are known in the art and can be used to carry out the present description.

Another aspect of the present description provides a method for detecting the presence of a test molecule that competes with an antibody of the present description to bind mesothelin. An example of such assay would involve detecting the amount of free antibody in a solution containing a certain amount of mesothelin in the presence or absence of the test molecule. An increase in the amount of free antibody (i.e., antibody that does not bind mesothelin) will indicate that the test molecule can compete with the antibody to bind mesothelin. In one embodiment, the antibody is labeled with a labeling group. Alternatively, the test molecule is labeled, and the amount of free test molecule is monitored in the presence or absence of the antibody.

The description also provides a detection kit for detecting mesothelin level. The kit includes an antibody that recognizes mesothelin protein, a lysis medium for dissolving samples, and general reagents and buffers required for detection, such as various buffers, detection markers, detection substrates, etc. The detection kit can be an *in vitro* diagnostic device.

The description specifically comprises an embodiment shown in any one of the following:
1. A mesothelin binding molecule, comprising an anti-mesothelin single domain antibody, wherein the complementarity determining region CDRs of the single domain antibody comprise CDR1, CDR2 and CDR3, wherein CDR1 comprises the sequence shown in SEQ ID NO: 1, CDR2 comprises the sequence shown in SEQ ID NO: 2, and CDR3 comprises the sequence shown in SEQ ID NO: 3, wherein,
   SEQ ID NO: 1 is X₁X₂X₃X₄X₅X₆X₇X₈X₉X₁₀X₁₁, wherein X₁ is G, E, T or A, X₂ is S, N, P, A, H, D, K, I or F, X₃ is I, S, V, T, D, L, A, M or H, X₄ is F, S, I, A, L or G, X₅ is N, S, G, A, D, T, E, R or H, X₆ is I, L, F, Y, E or N, X₇ is N, A, G, D, K, Y, L or S, X₈ is A, Y, V, D or N, X₉ is E or null, X₁₀ is F or null, X₁₁ is A or null,
   SEQ ID NO: 2 is X₁X₂X₃X₄X₅X₆X₇XₛX₉X₁₀X₁₁X₁₂, wherein X₁ is I, M, A, T or L, X₂ is S, G, N, D, T or V, X₃ is S, N, A, R, G or T, X₄ is S, T, G, D or N, X₅ is N, G, T or I, X₆ is S, R, N, D, K, T or G, X₇ is D, T, S, I or K, X₈ is N, T, G, D or null, X₉ is T, K, G or null, X₁₀ is G, R or null, X₁₁ is V, T or null, X₁₂ is T or null,
   SEQ ID NO: 3 is X₁X₂X₃X₄X₅X₆X₇X₈X₉X₁₀X₁₁X₁₂X₁₃X₁₄X₁₅X₁₆X₁₇X₁₈X₁₉X₂₀X₂₁X₂₂X₂₃, wherein X₁ is N, H, A or Q, X₂ is L, A, G or V, X₃ is S, R, E, G, D or T, X₄ is N, A, R, G, D, K, V, T, I, E or Q, X₅ is Y, F, K, S, C, I, D, G, H or R, X₆ is D, A, V, G, R, N, P, T, C, Q or S, X₇ is R, Y, T, A, D, K, L, E, S or G, X₈ is K, S, F, T, D, Q, E, G, P, R, Y or N, X₉ is D, G, I, H, S, Y, Q, K, T, R or V, X₁₀ is R, Y, H, N, T, D, P, V, K, C, L or S, X₁₁ is Y, D, E, P, F, S, L, I or null, X₁₂ is P, V, Q, A, Y, F, N, D, R or null, X₁₃ is D, A, Y, V, Q, P or null, X₁₄ is Y, L, P, A, E or null, X₁₅ is C, S, M, P, T or null, X₁₅ is C, S, M, P or null, X₁₆ is V, D, S, Y or null, X₁₇ is L, F, V, D or null, X₁₈ is R, G, M, S or null, X₁₉ is D, N, S or null, X₂₀ is Y, L or null, X₂₁ is Y or null, X₂₂ is A or null, X₂₃ is D or null.
2. The mesothelin binding molecule according to Item 1, wherein,
   SEQ ID NO: 1 is X₁X₂X₃X₄X₅X₆X₇X₈, wherein X₁ is G, E, T or A, X₂ is S, N, P, A, H, D, K, I or F, X₃ is I, S, V, T, D, L, A, M or H, X₄ is F, S, I, A, L or G, X₅ is N, S, G, A, D, T, E or H, X₆ is I, L, F, Y or N, X₇ is N, A, G, D, K, Y or S, X₈ is A, Y, V or N; preferably, SEQ ID NO: 1 is X₁X₂X₃X₄X₅X₆X₇X₈, wherein X₁ is G, E, T or A, X₂ is S, N, P, H or I, X₃ is I, S, T, D, L or A, X₄ is F, I or L, X₅ is S, T or E, X₆ is I, F or Y, X₇ is N, A, D or Y, X₈ is A or Y,
   SEQ ID NO: 2 is X₁X₂X₃X₄X₅X₆X₇XₛX₉X₁₀X₁₁X₁₂, wherein X₁ is I, M, A, T or L, X₂ is S, G, N, D, T or V, X₃ is S, N, A, R or T, X₄ is S, T, G, D or N, X₅ is N, G, T or I, X₆ is S, R, N, D, K, T or G, X₇ is D, T, S, I or K, X₈ is N, T, G, D or null, X₉ is T, K, G or null, X₁₀ is G, R or null, X₁₁ is V, T or null, X₁₂ is T or null; preferably, SEQ ID NO: 2 is X₁X₂X₃X₄X₅X₆X₇XₛX₉X₁₀X₁₁X₁₂, wherein X₁ is I, A or T, X₂ is S, G, N or T, X₃ is S or R, X₄ is T, G or D, X₅ is N, G or T, X₆ is S, R, N, K or T, X₇ is D, T, S, I or K, X₈ is N, G or null, X₉ is T, G or null, X₁₀ is G or null, X₁₁ is V or null, X₁₂ is T or null,
   SEQ ID NO: 3 is X₁X₂X₃X₄X₅X₆X₇X₈X₉X₁₀X₁₁X₁₂X₁₃X₁₄X₁₅X₁₆X₁₇X₁₈X₁₉X₂₀X₂₁X₂₂X₂₃, wherein X₁ is N, H, A or Q, X₂ is L, A, G or V, X₃ is S, R, E, G, D or T, X₄ is N, A, R, G, D, K, V, T, I or Q, X₅ is Y, F, K, S, C, I, D, G, H or R, X₆ is D, A, V, G, R, N, P, T, C, Q or S, X₇ is R, Y, T, A, D, K, L, E, S or G, X₈ is K, S, F, T, D, Q, E, G, P, R, Y or N, X₉ is D, G, I, H, S, Y, Q, K, T or V, X₁₀ is R, Y, H, N, T, D, P, V, K, C or S, X₁₁ is Y, D, E, P, F, S, L or null, X₁₂ is P, V, Q, A, Y, F, N, D or null, X₁₃ is D, A, Y, V, Q or null, X₁₄ is Y, L, P, A, E or null, X₁₅ is C, S, M, P or null, X₁₆ is V, D, S, Y or null, X₁₇ is L, F, V, D or null, X₁₈ is R, G, M, S or null, X₁₉ is D, N, S or null, X₂₀ is Y, L or null, X₂₁ is Y or null, X₂₂ is A or null, X₂₃ is D or null; preferably, SEQ ID NO: 3 is AX₂X₃X₄X₅X₆X₇X₈X₉X₁₀X₁₁X₁₂X₁₃X₁₄X₁₅X₁₆X₁₇X₁₈X₁₉, wherein X₂ is A or V, X₃ is S, E, G or D, X₄ is N, R, D or I, X₅ is Y, F, K, D or H, X₆ is D, G, P, C or S, X₇ is R, Y, D, K or L, X₈ is K, Q, G or N, X₉ is D, S, Y or Q, X₁₀ is R, Y, D, V or S, X₁₁ is Y, D, F, S, L or null, X₁₂ is P, F, or null, X₁₃ is D, V or null, X₁₄ is P or null, X₁₅ is S or null, X₁₆ is D or null, X₁₇ is F or null, X₁₈ is G or null, X₁₉ is N or null.
3. The mesothelin binding molecule according to Item 1 or 2, wherein CDR1 of the single domain antibody comprises the sequence shown in any one of SEQ ID NOs: 4-27 and 73, CDR2 comprises the sequence shown in any one of SEQ ID NOs: 28-47 and 74, and CDR3 comprises the sequence shown in any one of SEQ ID NOs: 48-72 and 75,
   preferably, the single domain antibody contains CDR1, CDR2 and CDR3 shown in any one of groups a1 to a26 in Table 1.
4. The mesothelin binding molecule according to Item 1 or 2, wherein,
   the FR region of the single domain antibody comprises the FR region of any VHH selected from SEQ ID NOs: 76-120, and/or
   the single domain antibody VHH is as shown in any one of SEQ ID NOs: 76-120, and/or
   the mesothelin binding molecule is a monovalent or multivalent single domain antibody, a multispecific single domain antibody, a heavy chain antibody or an antigen binding fragment thereof, an antibody or an antigen binding fragment thereof comprising one, two or more of the single domain antibodies.
5. The mesothelin binding molecule according to Item 4, wherein the mesothelin binding molecule is a heavy chain antibody, which further contains the heavy chain constant regions CH2 and CH3,
   preferably, the heavy chain constant region comprises the sequence shown in SEQ ID NO: 121.
6. A polynucleotide, wherein the polynucleotide comprises a sequence selected from:
   (1) a coding sequence of the mesothelin binding molecule according to any one of Items 1-5;
   (2) a complementary sequence of (1);
   (3) a 5-50bp fragment of any sequence of (1) or (2).
7. A nucleic acid construct, wherein the nucleic acid construct comprises the polynucleotide according to Item 6,
   preferably, the nucleic acid construct is a recombinant vector or expression vector.
8. A Phage comprising the mesothelin binding molecules according to any one of Items 1-5,
   preferably, the mesothelin binding molecule is displayed on the surface of the phage.
9. A host cell, wherein the host cell:
   (1) expresses the mesothelin binding molecules according to any one of Items 1-5; and/or
   (2) comprises the polynucleotide according to Item 6; and/or
   (3) comprises the nucleic acid construct according to Item 7.
10. A method for producing mesothelin binding molecules, comprising: culturing the host cell according to Item 9 under conditions suitable for producing the mesothelin binding molecules, and optionally purifying the mesothelin binding molecules from the culture.
11. A pharmaceutical composition comprising the mesothelin binding molecule according to any one of Items 1-5, the polynucleotide according to Item 6, the nucleic acid construct according to Item 7, the phage according to Item 8 or the host cell according to Item 9, and a pharmaceutically acceptable excipient,
   preferably, the pharmaceutical composition is used for treating cancer.
12. Use of the mesothelin binding molecule according to any one of Items 1-5 in the preparation of a medicament for the prevention or treatment of cancer.
13. A kit for detecting mesothelin or MD50, for use in evaluating the therapeutic effect of a medicament or diagnose cancer, the kit comprises the mesothelin binding molecule according to any one of Items 1-5, the polynucleotide according to Item 6, the nucleic acid construct according to Item 7, the phage according to Item 8 or the host cell according to Item 9,
   preferably, the kit further comprises a reagent for detecting the binding of mesothelin or MD50 to a single domain antibody, an antibody, or an antigen binding fragment thereof,
   more preferably, the reagent is a reagent that detects the binding by enzyme-linked immunosorbent assay.
14. A non-diagnostic method for detecting the presence of mesothelin or MD50 in a sample, the method comprises: incubating the mesothelin binding molecule according to any one of Items 1-5 with the sample and detecting the binding of mesothelin or MD50 to a single domain antibody, antibody, or antigen binding fragment thereof, thereby determining the presence of mesothelin or MD50 in the sample.
15. Use of the mesothelin binding molecule according to any one of Items 1-5 in the preparation of kits for detecting mesothelin or MD50 in samples, evaluating the therapeutic effect of a medicament, or diagnosing cancer.

The present description will be described below in the form of specific examples. It should be understood that these examples are merely illustrative and are not intended to limit the scope of the present description. Unless otherwise specified, the methods and materials used in the examples are conventional materials and methods in the art.

### Example

### Example 1. Immunization of alpaca

### 1.1 Preparation of immunogen:

The sequence of mesothelin protein was obtained from NCBI, and was fused with the sequence of human IgG Fc fragment, and the eukaryotic expression vector of pCDNA3.4 (Thermo) plasmid was synthesized and constructed by Nanjing Genscript Company. The synthesized plasmid was expressed using ExpiCHO^{™} (Thermo Fisher) expression system. After expression, 5 ml of protein A pre-packed column (GE) was used for one-step affinity purification, and the purified sample was replaced into PBS buffer. After the purity was identified by SDS-PAGE electrophoresis gel and HPLC, and the activity was identified by ELISA, the sample was split and frozen in -80°C refrigerator for subsequent immunization.

### 1.2 Immunization of Alpaca:

For first immunization, 400 µg of antigen (MSLN.hFc) was mixed with the adjuvant (GERBU FAMA). Alpaca was immunized subcutaneously at four sites on the back, and the amount of each site was 1 mL. For the second to sixth immunization, 200 µg of antigen was used. Alpacas were immunized by subcutaneous injection at four sites on the back, and the amount of immunization at each site was 1 mL. The interval between immunizations was one week.

### 1.3 Detection of immune serum titer:

### 1.3.1 Detection of titer at protein level

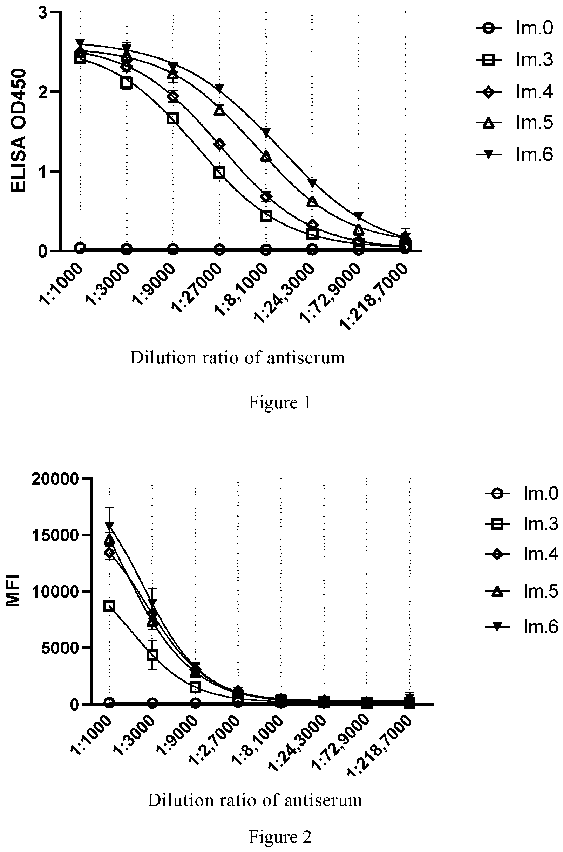

MSLN.His antigen was coated overnight at 4 degrees. After blocking and washing, the gradient diluted serum was added to the ELISA plate for incubation, and then incubated with the anti-llama (anti-Alpaca) IgG HRP (Abcam) antibody. After washing, TMB chromogenic solution was added for development, and the reaction was terminated with 2 M HCl. Then the absorbance value at OD450 nm was detected with a microplate reader. As shown in Figure 1, the titer of Alpaca reached a higher level (>243000) after six immunizations.

### 1.3.2 Detection of titer at cell level

MSLN transfected HEK293T cells were plated in 96 well plates with a cell volume of 3 × 10⁵ cells/well. The cells were then incubated with 3-fold gradient diluted serum. After incubation and washing, anti-llama IgG PE (Jackson) antibody was added for incubation. After washing, the cells were resuspended with PBS, and then the fluorescence intensity (MFI) was detected by flow cytometry (Beckman). The results were shown in Figure 2.

### Example 2. Construction and screening of nanobody immune library for MSLN

(1) After six immunizations, 100 mL of camelid peripheral blood lymphocytes were extracted, and total RNA was extracted. RNA was extracted according to the instructions of RNAiso reagent of Takara.
(2) The first strand of cDNA was synthesized with RNA as template and oligo dT as primer according to the instructions of reverse transcriptase of Takara.
(3) The variable region coding gene of heavy chain antibody was obtained by nested PCR using PrimeSTAR high fidelity DNA polymerase. The variable region fragment of the heavy chain antibody was amplified by nested PCR:
   First round of PCR:
      Upstream primer: GTCCTGGCTGCTCTTCTACAAGGC
      Downstream primer: GGTACGTGCTGTTGAACTGTTCC
      The fragment between the heavy chain antibody guide peptide and antibody CH2 was amplified, annealed at 55°C for 30 cycles; a DNA fragment of about 600 bp was recovered as a template for the second round of PCR.
   Second round of PCR:
      Upstream primer: GATGTGCAGCTGCAGGAGTCTGGRGGAGG
      Downstream primer: GGACTAGTGCGGCCGCTGGAGACGGTGACCTGGGT
      The fragment (long fragment and short fragment) between the FR1 region and the long and short hinge regions of the heavy chain antibody was amplified, annealed at 55°C for 30 cycles, and the target fragment was recovered. The result showed that the size of the fragment was about 500 bp, that is, the nanoantibody gene electrophoresis band was about 500 bp.
(4) The phage pME207 and PCR amplification products were digested with Sfi I and Not I (NEB), respectively. After recovery and quantification, the two fragments were ligated with T4 DNA ligase (Takara) at a molar ratio of 1:3 and ligated overnight at 16°C.
(5) After ethanol precipitation, the ligated product was dissolved in 100 µL sterile water and electroporated into *Escherichia coli* TG1 in ten times. 100µL of the bacterial solution was taken after electric shock and culture, diluted by multiple ratio, coated on an ampicillin LB culture plate, the storage capacity was calculated, and the rest was coated with ampicillin 2 × YT culture plate, at 37°C, invertly cultured for 13-16 h. After scraping and washing the colonies on the culture plate with 10 ml, 2 × YT medium, 25% glycerol at the final concentration was added, split, and stored at -80°C for further use. The size of the storage capacity is 4.3 × 10⁹. To detect the insertion rate of the library, 48 clones were randomly selected for colony PCR, and the results showed that the insertion rate had reached more than 90%.
(6) According to the calculated library capacity results, viable cells with 10 times the library capacity were seeded in 200ml of 2 × YT (containing 2% glucose, 100 µg/ml ampicillin), cultured at 37°C for 200 r/min until the OD600 reached 0.5, auxiliary phage was added according to the multiplicity of infection of 20:1, and left for 30 min at 37°C, 200 r/min for 30 min. The culture was centrifuged, and the pellet was resuspended with 200 ml of 2 × YT (containing 100 µg/ml ampicillin and 50 µg/ml kanamycin), incubated overnight at 37°C, 250 r/min, centrifuged at 8000rpm to obtain the supernatant, added with 5 × PEG/NaCl solution, placed on ice for 60 min, centrifuged at 8000rpm for 30 min. The pellet was resuspended in 5 ml of PBS to obtain the single domain heavy chain antibody (VHH) immune library against MSLN, and 10 µL was taken to determine the titer, and the rest were split at -80°C for storage.
(7) MD50, a protein with 94 amino acids (GenBank: AAH09272.1, 487-580) at the proximal membrane end of mesothelin, was coated on an ELISA plate at 10 µg/ml, 100 µl per well, and placed overnight at 4 °C. At the same time, a negative control was set up. The next day, 200 µL, 3%BSA were added to the five wells and blocked for 2 hours at room temperature. Two hours later, they were washed three times with PBST (containing 0.05% Tween 20 in PBS). After the plate was washed, 100 µL of phage pre-blocked with 5% skim milk (2-3 × 10¹¹ tfu immunized camelid nanoantibody phage display gene library) was added, left for 1.5 hours at room temperature, and then the supernatant after negative screening was transferred to the target antigen coated well for 1.5 hours at room temperature. It was washed with PBST (containing 0.05% Tween 20 in PBS) for 12 times to wash out the unbound phage. The phage specifically bound to MSLN was dissociated with Glycine (SIGMA), and the eluted phage was neutralized by Tris (Invitrogen, 1 M, pH 8.0) and infected with TG1 in logarithmic phase. After propagation and expansion, the next round of "adsorption-elution" was carried out. Finally, TG1 was impregnated with the eluted phage, and the expression of nanoantibody was induced by IPTG (Thermo). The ELISA plate was coated with MSLN protein and MD50 protein. The supernatant was taken for ELISA detection, and the clones with OD450>0.5 were picked for sequencing.
(8) After sequence analysis, a total of 25 clones were obtained that could bind MSLN protein and MD50 protein, and one clone that could bind MSLN.His protein (Sino Biological, Inc., MSN-H5223), as shown in the table below. The amino acid sequence of the negative control is shown in SEQ ID NO: 2 in CN106046152A.

| Clone No. | ELISA (OD450) | | |
|---|---|---|---|
| | MSLN.Fc protein | MD50 protein | Negative control |
| M001 | 1.7205 | 1.3526 | 0.0184 |
| M003 | 2.3825 | 1.7271 | 0.0104 |
| M004 | 1.1035 | 1.8141 | 0.0085 |
| M005 | 1.1845 | 1.0257 | 0.0072 |
| M010 | 2.7475 | 2.0856 | 0.007 |
| M012 | 0.9383 | 2.0471 | 0.0075 |
| M032 | 1.412 | 1.7154 | 0.0076 |
| M044 | 2.2013 | 2.0356 | 0.0083 |
| M047 | 1.981 | 2.0705 | 0.018 |
| M050 | 2.717 | 0.5122 | 0.0099 |
| M118 | 2.0519 | 2.0647 | 0.0074 |
| M119 | 2.4918 | 1.4572 | 0.0071 |
| M126 | 1.9473 | 0.926 | 0.0068 |
| M129 | 2.3985 | 1.6601 | 0.007 |
| M132 | 1.3363 | 1.1699 | 0.007 |
| M137 | 1.2739 | 2.088 | 0.0087 |
| M143 | 1.622 | 1.298 | 0.0085 |
| M156 | 1.667 | 1.5335 | 0.0074 |
| M169 | 1.3582 | 1.1924 | 0.007 |
| M182 | 1.0259 | 1.1372 | 0.0075 |
| M191 | 1.6945 | 1.7099 | 0.0074 |
| M197 | 1.2077 | 1.6899 | 0.0074 |
| M210 | 1.9525 | 0.8227 | 0.0071 |
| M224 | 1.4472 | 1.7766 | 0.0074 |
| M344 | 2.2154 | 2.0837 | 0.0082 |

| Clone No. | ELISA (OD450) | |
|---|---|---|
| | MSLN. His protein | Negative control |
| M2339 | 2.1478 | 0.0479 |

### Example 3. Expression and purification of candidate antibodies

The nanoantibodies were constructed on pCDNA3.4-IgG4 vector, and then expressed by ExpiCHOTM (Thermo Fisher) expression system. After one week of expression, the supernatant was collected for protein A (GE) purification. Then Nanodrop was used to detect the protein concentration, and HPLC was used to detect the protein purity. The purity and yield of the obtained protein met the requirment of subsequent tests.

### Example 4. Characterization of candidate antibodies

(1) Detection of affinity at protein level: the binding kinetics and affinity of heavy chain antibody to human MSLN.His antigen were determined using surface plasmon resonance (SPR). The purified antibody was flowed through the sensor chips pre-immobilized with protein A, and the antibody was captured by protein A. Then five different concentrations of MSLN.His protein were used as the mobile phase, and the binding time and dissociation time were 30min and 60min, respectively. Biacore Evaluation Software 2.0 (GE) was used to analyze the binding rate (kon, or ka), dissociation rate (koff, or kd) and equilibrium constant (KD). The rabbit antibody YP218, which binds to segment III of mesothelin, was selected as a positive control (Zhang et al., Sci Rep 2015, 21;5:9928). The results are shown in the table below.

| Antibody | Kₒₙ (1/Ms) | K_{off} (1/s) | KD (M) |
|---|---|---|---|
| M003-IgG4 | 8.82E+05 | 4.29E-04 | 4.90E-10 |
| M044-IgG4 | 5.37E+04 | 7.50E-05 | 2.10E-09 |
| M118-IgG4 | 6.79E+05 | 5.05E-04 | 7.40E-10 |
| M119-IgG4 | 8.26E+05 | 9.58E-05 | 1.20E-10 |
| M129-IgG4 | 1.40E+06 | 5.37E-05 | 4.00E-11 |
| M156-IgG4 | 5.92E+05 | 5.40E-05 | 9.00E-11 |
| M344-IgG4 | 3.59E+05 | 2.35E-05 | 7.00E-11 |
| Other heavy chain antibodies | 5.48E+04 to 1.25E+06 | 2.01E-03 to 8.73E-05 | 4.21E-9 to 2.34E-11 |
| YP218-IgG4 | 4.02E+04 | 1.23E-04 | 3.10E-09 |
| M2339-IgG4 | 6.90E+04 | 1.82E-05 | 2.64E-10 |

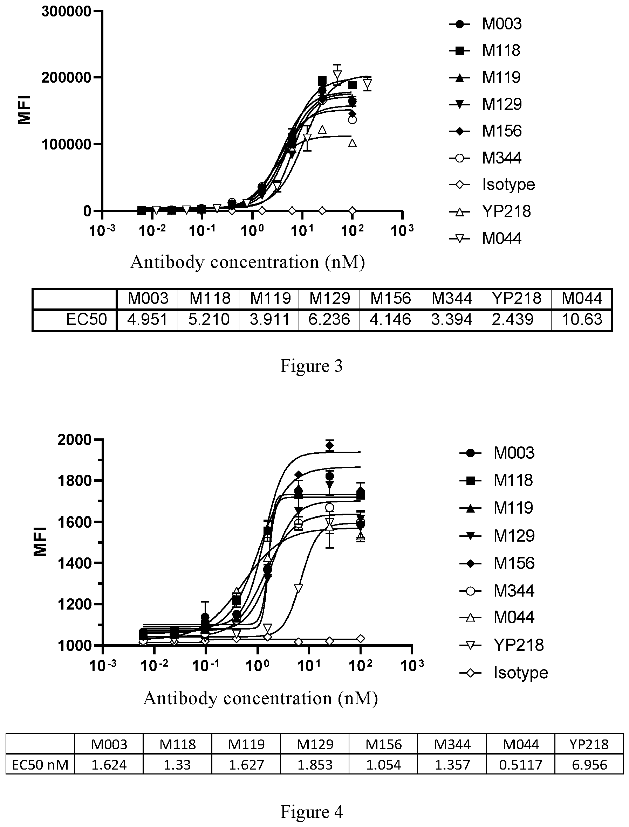

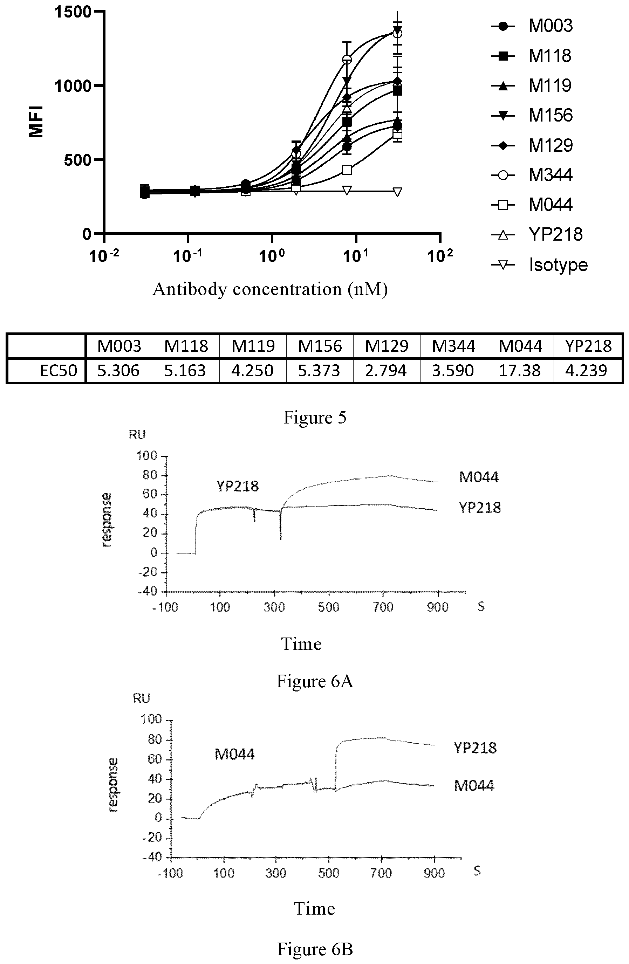

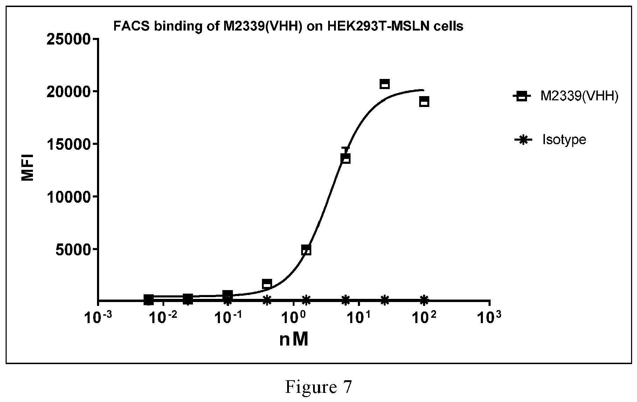

(2) Detection of affinity at cell level: HEK293T cells that express segment III of MSLN were plated in 96 well plates with 3 × 10⁵ cells/well, and then HEK293T MSLN-III cells were incubated with gradient diluted heavy chain antibody. After incubation for half an hour, anti-human IgG PE (Jackson Immuno Research, Code:109-117-008, Lot:145501) as the secondary antibody for detection was added for incubation, and then detected with CytoFLEX flow cytometer. The EC50 of the antibody was calculated by fitting the curve. Isotype is an isotype control (negative control), and its amino acid sequence is shown in SEQ ID NO: 2 in CN106046152A. The results were shown in Figures 3 and 7. The EC50 of the remaining heavy chain antibodies was 3.637-171.4 nM.
(3) Detection of binding of tumor cells to MSLN: SKOV3 and Aspc-1 tumor cells expressing MSLN were respectively plated in 96 well plates with 3 × 10⁵ cells/well, and then the tumor cells were incubated with gradient diluted heavy chain antibodies. After incubation on ice for half an hour, anti-human IgG PE (Jackson Immuno Research, Code:109-117-008, Lot:145501) as the secondary antibody for detection was added for incubation, and then CytoFLEX flow cytometer was used for detection. The EC50 of the antibody was calculated by fitting the curve. "Isotype" was isotype control (negative control). The results are shown in Figures 4 and 5. The EC50 of the other heavy chain antibodies with SKOV3 tumor cell line was 0.429-10.253 nM, and that with AsPC-1 tumor cell line was 2.337-31.764 nM.

### Example 5. Epitope competition experiment

The instrument Biacore T200 (GE), detection temperature of 25 °C, buffer of HBS-EP+(10 mM HEPES, 150 mM NaCl, 3 mM EDTA and 0.05% v/v Surfactant P20, GE) at a flow rate of 30 ul/min was used. MSLN-His was dissolved in acetate buffer, pH4.0 (Biacore Amine Coupling Kit, GE), to a final concentration of 10 ug/ml. According to the product instructions of amino coupling kit, MSLN His was fixed to CM5 chip (GE) channel 2 (FC-2), about 21 RU. Channel 1 (FC-1) was used as blank control. M044 and YP218 antibodies are diluted with 1 × HBS-EP+ to the target concentration, respectively, one antibody was injected to the above MSLN fixed chips FC-1 and FC-2 to the saturation level, and then the other antibody was injected to the saturation level to evaluate the competition relationship between the two antibodies. After the second antibody injection, the complex was dissociated for 400s. Finally, 50 mM NaOH was injected for 15s to regenerate the surface of the chip. Biacore Evaluation Software 2.0 (GE) was used for data processing, dual reference subtraction was used for sensorgrams, and FC2-1 signals were recorded. The results are shown in Figure 6a and Figure 6B. The results showed that the binding epitopes of M044 and YP218 antibodies were completely different. The relationship between the remaining nanoantibodies and the epitope of YP218 was as follows.

| Clone No. | Relationship with YP218 epitope |
|---|---|
| M001 | absolutely different |
| M003 | absolutely different |
| M004 | absolutely different |
| M005 | partly overlapping |
| M010 | partly overlapping |
| M012 | absolutely different |
| M032 | partly overlapping |
| M044 | absolutely different |
| M047 | absolutely different |
| M050 | partly overlapping |
| M118 | absolutely different |
| M119 | absolutely different |
| M126 | partly overlapping |
| M129 | absolutely different |
| M132 | absolutely different |
| M137 | partly overlapping |
| M143 | partly overlapping |
| M156 | absolutely different |
| M169 | partly overlapping |
| M182 | partly overlapping |
| M191 | absolutely different |
| M197 | partly overlapping |
| M210 | absolutely different |
| M224 | absolutely different |
| M344 | absolutely different |

### Example 6. Antibody humanization

Referring to the method of humanizing nanoantibodies (J. Biol. Chem. 2009; 284: 3273-3284), M2339 was humanized using the method of CDR region transplantation. In IgBLAST (http://www.ncbi.nlm.nih.gov/igblast/), germline with high homology with M2339 was selected as the template in the database. The humanized nanoantibodies shown in the table below were obtained. Biacore was used to detect the affinity of the antibody before and after humanization. The purified antibody was flowed through the sensor chip pre-fixed with protein A, and the antibody was captured by protein A. Then five different concentrations of MSLN.His protein were used as the mobile phase, and the association time and dissociation time were 30 min and 60 min, respectively. The binding rate (ka), dissociation rate (kd), and equilibrium constant (kd) were analyzed using Biacore Evaluation Software 2.0 (GE).

| Antibody No. | kₐ (1/Ms) | k_{d}(1/s) | KD (M) |
|---|---|---|---|
| M2339-z11-1 | 1.02E+05 | 6.08E-03 | 5.95E-08 |
| M2339-z11-2 | 6.23E+04 | 1.74E-05 | 2.79E-10 |
| M2339-z11-3 | 4.74E+04 | 1.32E-04 | 2.78E-09 |
| M2339-z11-4 | 8.00E+04 | 1.19E-03 | 1.49E-08 |
| M2339-z11-5 | 2.33E+05 | 8.78E-03 | 3.77E-08 |
| M2339-z11-6 | 1.74E+05 | 8.24E-03 | 4.74E-08 |
| M2339-z11-7 | 3.98E+04 | 5.18E-05 | 1.30E-09 |
| M2339-z11-8 | 4.43E+04 | 1.60E-05 | 3.61E-10 |
| M2339-z11-9 | 8.38E+04 | 1.20E-05 | 1.43E-10 |
| M2339-z11-10 | 4.10E+04 | 9.81E-06 | 2.39E-10 |
| M2339-z11-11 | 4.30E+04 | 1.18E-05 | 2.74E-10 |
| M2339-z11-12 | 8.38E+04 | 1.02E-03 | 1.22E-08 |
| M2339-z11-13 | 6.52E+04 | 9.29E-06 | 1.42E-10 |
| M2339-z11-14 | 4.44E+04 | 1.01E-05 | 2.28E-10 |
| M2339-z11-15 | 7.10E+04 | 1.14E-05 | 1.60E-10 |
| M2339-z11-16 | 6.78E+04 | 6.52E-06 | 9.61E-11 |
| M2339-z11-17 | 3.93E+04 | 1.56E-04 | 3.98E-09 |
| M2339-z1-1 | 3.26E+02 | 7.39E-04 | 2.27E-06 |
| M2339-z1-2 | / | / | / |

### Example 7. Tissue cross-reaction

34 tissues were selected for frozen section, dried at room temperature, and fixed with acetone. Blocking was performed using Reagent A and Reagent B of IHC Biotin Block Kit (Sangon, E674001). Biotin labeled antibody samples were incubated for 30min, and horseradish peroxidase labeled streptavidin (Abcam, ab7403) was added for incubation for 15min after washing. DAB development, hematoxylin counterstaining, neutral plastic sealing, natural air drying, and then microscopic examination were performed. The positive control was anti-mesothelin antibody (Biotin) (ab271813), and the negative control was biotin labeled IgG4 isotype control. The table below shows the tissue cross-reaction results of M044, YP218 and M2339-z11-2. Most of the other nano-antibodies (except M2339 related antibodies shown in Table 2) had the same binding properties with M044 on human normal tissues, showing specific binding; wherein five of the antibodies showed nonspecific binding, which were M010, M032, M050, M137, M169, M182, which mainly bound to the cell membrane of neuronal cells and nerve fibers. The other M2339 related antibodies showed the same binding properties with M2339-z11-2 on human normal tissues.

| **No.** | **Organ** | **Positive control** | **Negative control** | **M044.bioti n** | **YP218.bioti n** | **bio-M2339-z11-2** |
|---|---|---|---|---|---|---|
| 1 | tonsil | positivity in partial epithelial cell | - | positivity in occasional epithelium | positivity in partial epithelial cell | positivity **in** partial epithelial cell |
| 2 | liver | - | - | - | - | |
| 3 | thyroid | - | - | - | - | |
| 4 | ileum | positivity in | - | Very | positivity in | positivity in |
| | | partial lamina muscularis | | occasional positivity in matrix | partial lamina muscularis epithelial cell | partial lamina muscularis |
| 5 | cerebellum | positivity in granulose layer (5%) | - | - | - | |
| 6 | spleen | | - | - | - | |
| 7 | heart | - | - | - | - | |
| 8 | artery | - | - | - | - | |
| 9 | parathyroi d | - | - | - | - | |
| 10 | Spinal cord (without cutting to nerve fibers) | positivity in few cells (2%) | - | - | - | |
| 11 | oviduct | positivity in epithelium | - | - | positivity in oviductal epithelial cell | strong positivity in oviductal epithelial cell (more than 75%) |
| 12 | duodenum | - | - | - | - | |
| 13 | pituitary | - | - | - | - | |
| 14 | stomach | - | - | - | - | |
| 15 | Testis | positivity in occasional seminiferou s epithelium | - | - | - | |
| 16 | Colon | positivity in occasional mucosa epithelium, positivity in partial lamina muscularis | - | - | positivity in partial lamina muscularis epithelial cell | positivity in partial lamina muscularis |
| 17 | Bladder | positivity in occasionally positive transitional epithelium | - | - | - | positivity in occasional epithelium |
| 18 | Corpus | - | - | - | - | |
| 19 | lymph node | - | - | - | - | |
| 20 | Skin | - | - | - | - | |
| 21 | Placenta | Poor positivity | - | - | - | |
| 22 | Eye | - | - | - | - | positivity in epithelium |
| 23 | Pancreas | - | - | - | - | |
| 24 | Kidney | - | - | - | - | |
| 25 | Breast | - | - | - | - | |
| 26 | adrenal gland | - | - | - | - | |
| 27 | Vein | - | - | - | - | |
| 28 | Ureter | - | - | - | | |
| 29 | cervix | - | - | - | - | |
| 30 | Prostate | positivity in occasional glandular cells | - | - | - | |
| 31 | Ovary | positivity in occasional cortical area | - | - | - | |
| 32 | skeletal muscle | - | - | - | - | |
| 33 | Lung | positivity in occasional bronchial area (2-5%) | - | - | - | |
| 34 | Brain | few positivity (2%) | - | - | - | |

## Claims

1. A mesothelin binding molecule, comprising an anti-mesothelin single domain antibody, wherein the complementarity determining region (CDR) of the single domain antibody comprises CDR1, CDR2 and CDR3, and CDR1 comprises the sequence shown in SEQ ID NO: 1, CDR2 comprises the sequence shown in SEQ ID NO: 2, and CDR3 comprises the sequence shown in SEQ ID NO: 3, wherein,
SEQ ID NO: 1 is X₁X₂X₃X₄X_{S}X₆X₇X₈X₉X₁₀X₁₁, wherein X₁ is G, E, T or A, X₂ is S, N, P, A, H, D, K, I or F, X₃ is I, S, V, T, D, L, A, M or H, X₄ is F, S, I, A, L or G, X₅ is N, S, G, A, D, T, E, R or H, X₆ is I, L, F, Y, E or N, X₇ is N, A, G, D, K, Y, L or S, X₈ is A, Y, V, D or N, X₉ is E or null, X₁₀ is F or null, X₁₁ is A or null,
SEQ ID NO: 2 is X₁X₂X₃X₄X₅X₆X₇XₛX₉X₁₀X₁₁X₁₂, wherein X₁ is I, M, A, T or L, X₂ is S, G, N, D, T or V, X₃ is S, N, A, R, G or T, X₄ is S, T, G, D or N, X₅ is N, G, T or I, X₆ is S, R, N, D, K, T or G, X₇ is D, T, S, I or K, X₈ is N, T, G, D or null, X₉ is T, K, G or null, X₁₀ is G, R or null, X₁₁ is V, T or null, X₁₂ is T or null,
SEQ ID NO: 3 is X₁X₂X₃X₄X₅X₆X₇X₈X₉X₁₀X₁₁X₁₂X₁₃X₁₄X₁₅X₁₆X₁₇X₁₈X₁₉X₂₀X₂₁X₂₂X₂₃, wherein X₁ is N, H, A or Q, X₂ is L, A, G or V, X₃ is S, R, E, G, D or T, X₄ is N, A, R, G, D, K, V, T, I, E or Q, X₅ is Y, F, K, S, C, I, D, G, H or R, X₆ is D, A, V, G, R, N, P, T, C, Q or S, X₇ is R, Y, T, A, D, K, L, E, S or G, X₈ is K, S, F, T, D, Q, E, G, P, R, Y or N, X₉ is D, G, I, H, S, Y, Q, K, T, R or V, X₁₀ is R, Y, H, N, T, D, P, V, K, C, L or S, X₁₁ is Y, D, E, P, F, S, L, I or null, X₁₂ is P, V, Q, A, Y, F, N, D, R or null, X₁₃ is D, A, Y, V, Q, P or null, X₁₄ is Y, L, P, A, E or null, X₁₅ is C, S, M, P, T or null, X₁₅ is C, S, M, P or null, X₁₆ is V, D, S, Y or null, X₁₇ is L, F, V, D or null, X₁₈ is R, G, M, S or null, X₁₉ is D, N, S or null, X₂₀ is Y, L or null, X₂₁ is Y or null, X₂₂ is A or null, X₂₃ is D or null.

2. The mesothelin binding molecule according to claim 1, wherein,
SEQ ID NO:1 is X₁X₂X₃X₄X₅X₆X₇X₈, wherein X₁ is G, E, T or A, X₂ is S, N, P, A, H, D, K, I or F, X₃ is I, S, V, T, D, L, A, M or H, X₄ is F, S, I, A, L or G, X₅ is N, S, G, A, D, T, E or H, X₆ is I, L, F, Y or N, X₇ is N, A, G, D, K, Y or S, X₈ is A, Y, V or N; preferably, SEQ ID NO: 1 is X₁X₂X₃X₄X₅X₆X₇X₈, wherein X₁ is G, E, T or A, X₂ is S, N, P, H or I, X₃ is I, S, T, D, L or A, X₄ is F, I or L, X₅ is S, T or E, X₆ is I, F or Y, X₇ is N, A, D or Y, X₈ is A or Y,
SEQ ID NO: 2 is X₁X₂X₃X₄X₅X₆X₇XₛX₉X₁₀X₁₁X₁₂, wherein X₁ is I, M, A, T or L, X₂ is S, G, N, D, T or V, X₃ is S, N, A, R or T, X₄ is S, T, G, D or N, X₅ is N, G, T or I, X₆ is S, R, N, D, K, T or G, X₇ is D, T, S, I or K, X₈ is N, T, G, D or null, X₉ is T, K, G or null, X₁₀ is G, R or null, X₁₁ is V, T or null, X₁₂ is T or null; preferably, SEQ ID NO:2 is X₁X₂X₃X₄X₅X₆X₇XₛX₉X₁₀X₁₁X₁₂, wherein X₁ is I, A or T, X₂ is S, G, N or T, X₃ is S or R, X₄ is T, G or D, X₅ is N, G or T, X₆ is S, R, N, K or T, X₇ is D, T, S, I or K, X₈ is N, G or null, X₉ is T, G or null, X₁₀ is G or null, X₁₁ is V or null, X₁₂ is T or null,
SEQ ID NO: 3 is X₁X₂X₃X₄X₅X₆X₇X₈X₉X₁₀X₁₁X₁₂X₁₃X₁₄X₁₅X₁₆X₁₇X₁₈X₁₉X_{2O}X₂₁X₂₂X₂₃, wherein X₁ is N, H, A or Q, X₂ is L, A, G or V, X₃ is S, R, E, G, D or T, X₄ is N, A, R, G, D, K, V, T, I or Q, X₅ is Y, F, K, S, C, I, D, G, H or R, X₆ is D, A, V, G, R, N, P, T, C, Q or S, X₇ is R, Y, T, A, D, K, L, E, S or G, X₈ is K, S, F, T, D, Q, E, G, P, R, Y or N, X₉ is D, G, I, H, S, Y, Q, K, T or V, X₁₀ is R, Y, H, N, T, D, P, V, K, C or S, X₁₁ is Y, D, E, P, F, S, L or null, X₁₂ is P, V, Q, A, Y, F, N, D or null, X₁₃ is D, A, Y, V, Q or null, X₁₄ is Y, L, P, A, E or null, X₁₅ is C, S, M, P or null, X₁₆ is V, D, S, Y or null, X₁₇ is L, F, V, D or null, X₁₈ is R, G, M, S or null, X₁₉ is D, N, S or null, X₂₀ is Y, L or null, X₂₁ is Y or null, X₂₂ is A or null, X₂₃ is D or null; preferably, SEQ ID NO: 3 is AX₂X₃X₄X₅X₆X₇X₈X₉X₁₀X₁₁X₁₂X₁₃X₁₄X₁₅X₁₆X₁₇X₁₈X₁₉, wherein X₂ is A or V, X₃ is S, E, G or D, X₄ is N, R, D or I, X₅ is Y, F, K, D or H, X₆ is D, G, P, C or S, X₇ is R, Y, D, K or L, X₈ is K, Q, G or N, X₉ is D, S, Y or Q, X₁₀ is R, Y, D, V or S, X₁₁ is Y, D, F, S, L or null, X₁₂ is P, F, or null, X₁₃ is D, V or null, X₁₄ is P or null, X₁₅ is S or null, X₁₆ is D or null, X₁₇ is F or null, X₁₈ is G or null, X₁₉ is N or null.

3. The mesothelin binding molecule according to claim 1 or 2, wherein CDR1 of the single domain antibody comprises the sequence shown in any one of SEQ ID NOs: 4-27 and 73, CDR2 of the single domain antibody comprises the sequence shown in any one of SEQ ID NOs: 28-47 and 74, and CDR3 of the single domain antibody comprises the sequence shown in any one of SEQ ID NOs: 48-72 and 75,
preferably, the single domain antibody comprises CDR1, CDR2, and CDR3 shown in any one of the following groups a1 to a26:
| Group | CDR1 | CDR2 | CDR3 |
|---|---|---|---|
| a1 | 4 | 28 | 48 |
| a2 | 5 | 29 | 49 |
| a3 | 6 | 30 | 50 |
| a4 | 7 | 31 | 51 |
| a5 | 8 | 32 | 52 |
| a6 | 9 | 33 | 53 |
| a7 | 10 | 34 | 54 |
| a8 | 11 | 35 | 55 |
| a9 | 12 | 36 | 56 |
| a10 | 13 | 32 | 57 |
| a11 | 14 | 37 | 58 |
| a12 | 15 | 38 | 59 |
| a13 | 16 | 31 | 60 |
| a14 | 17 | 39 | 61 |
| a15 | 18 | 40 | 62 |
| a16 | 19 | 41 | 63 |
| a17 | 20 | 42 | 64 |
| a18 | 21 | 43 | 65 |
| a19 | 22 | 32 | 66 |
| a20 | 13 | 32 | 67 |
| a21 | 23 | 44 | 68 |
| a22 | 24 | 45 | 69 |
| a23 | 25 | 46 | 70 |
| a24 | 26 | 47 | 71 |
| a25 | 27 | 40 | 72 |
| a26 | 73 | 74 | 75 |

4. The mesothelin binding molecule according to claim 1 or 2, wherein,
the FR region of the single domain antibody comprises the FR region of any VHH selected from SEQ ID NOs: 76-120, and/or
the single domain antibody VHH is as shown in any one of SEQ ID NOs: 76-120, and/or
the mesothelin binding molecule is a monovalent or multivalent single domain antibody, a multispecific single domain antibody, a heavy chain antibody or an antigen binding fragment thereof, an antibody or an antigen binding fragment thereof comprising one, two or more of the single domain antibodies.

5. The mesothelin binding molecule according to claim 4, wherein the mesothelin binding molecule is a heavy chain antibody, which further comprises heavy chain constant regions CH2 and CH3,
preferably, the heavy chain constant regions comprise the sequence shown in SEQ ID NO: 121.

6. A polynucleotide, wherein the polynucleotide is selected from:
(1) a coding sequence of the mesothelin binding molecule according to any one of claims 1-5;
(2) a complementary sequence of (1);
(3) a 5-50 bp fragment of any sequence of (1) or (2).

7. A nucleic acid construct, wherein the nucleic acid construct comprises the polynucleotide of claim 6,
preferably, the nucleic acid construct is a recombinant vector or expression vector.

8. A phage comprising the mesothelin binding molecule according to any one of claims 1-5,
preferably, the mesothelin binding molecule is displayed on the surface of the phage.

9. A host cell, wherein the host cell:
(1) expresses the mesothelin binding molecule according to any one of claims 1-5; and/or
(2) comprises the polynucleotide according to claim 6; and/or
(3) comprises the nucleic acid construct according to claim 7.

10. A method for producing mesothelin binding molecule, comprising: culturing the host cell according to claim 9 under conditions suitable for producing the mesothelin binding molecule, and optionally purifying the mesothelin binding molecule from the culture.

11. A pharmaceutical composition, comprising the mesothelin binding molecule according to any one of claims 1-5, the polynucleotide according to claim 6, the nucleic acid construct according to claim 7, the phage according to claim 8 or the host cell according to claim 9, and a pharmaceutically acceptable excipient,
preferably, the pharmaceutical composition is used for treating cancer.

12. Use of the mesothelin binding molecule according to any one of claims 1-5 in preparing the medicament for preventing or treating a cancer.

13. A kit for detecting mesothelin or MD50, which is used to evaluate the therapeutic effect of a medicament or diagnose cancer, wherein the kit comprises the mesothelin binding molecule according to any one of claims 1-5, the polynucleotide according to claim 6, the nucleic acid construct according to claim 7, the phage according to claim 8 or the host cell according to claim 9,
preferably, the kit further comprises a reagent for detecting the binding of mesothelin or MD50 to a single domain antibody, an antibody, or an antigen binding fragment thereof,
more preferably, the reagent is a reagent that detects the binding by enzyme-linked immunosorbent assay.

14. A non-diagnostic method for detecting the presence of mesothelin or MD50 in a sample, wherein the method comprises: incubating the mesothelin binding molecule according to any one of claims 1-5 with the sample, and detecting the binding of mesothelin or MD50 to a single domain antibody, antibody, or antigen binding fragment thereof, thereby determining the presence of mesothelin or MD50 in the sample.

15. Use of the mesothelin binding molecule according to any one of claims 1-5 in preparing a kit for detecting mesothelin or MD50 in a sample, evaluating the therapeutic effect of a medicament or diagnosing a cancer.
